(19) Europäisches Patentamt
European Patent Office
Office européen des brevets

(11) EP 4 684 788 A1

(12) **EUROPEAN PATENT APPLICATION**
published in accordance with Art. 153(4) EPC

(43) Date of publication:
**28.01.2026 Bulletin 2026/05**

(21) Application number: **24774257.0**

(22) Date of filing: **22.03.2024**

(51) International Patent Classification (IPC):
***A61K 31/704*** *(2006.01)* ***A61K 31/506*** *(2006.01)*
***C07D 405/14*** *(2006.01)* ***A61P 35/00*** *(2006.01)*

(52) Cooperative Patent Classification (CPC):
**A61K 31/506; A61K 31/704; A61P 35/00; C07D 405/14**

(86) International application number:
**PCT/CN2024/083301**

(87) International publication number:
**WO 2024/193693 (26.09.2024 Gazette 2024/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **22.03.2023 CN 202310287072**
**19.12.2023 CN 202311750151**

(71) Applicants:
- **Ascentage Pharma (Suzhou) Co., Ltd.**
  **Suzhou, Jiangsu 215127 (CN)**
- **Ascentage Pharma Group Corp Limited**
  **Hong Kong (CN)**

(72) Inventors:
- **YU, Zhou**
  **Suzhou, Jiangsu 215127 (CN)**
- **YAO, Xinyi**
  **Suzhou, Jiangsu 215127 (CN)**
- **XIONG, Yan**
  **Suzhou, Jiangsu 215127 (CN)**
- **LIANG, Zhiyan**
  **Suzhou, Jiangsu 215127 (CN)**
- **ZHAI, Yifan**
  **Suzhou, Jiangsu 215127 (CN)**
- **YANG, Dajun**
  **Suzhou, Jiangsu 215127 (CN)**
- **TU, Yanhua**
  **Suzhou, Jiangsu 215127 (CN)**

(74) Representative: **AWA Denmark A/S**
**Strandgade 56**
**1401 Copenhagen K (DK)**

(54) **PHARMACEUTICAL COMPOSITION AND USE THEREOF**

(57) A pharmaceutical composition and the use thereof. Specifically, disclosed is a pharmaceutical composition, comprising: a substance X, which is a compound as represented by formula (I), a pharmaceutically acceptable salt thereof, a solvate thereof, or a solvate of the pharmaceutically acceptable salt thereof; and a substance Y, which is doxorubicin, a pharmaceutically acceptable salt thereof, a solvate thereof, or a solvate of the pharmaceutically acceptable salt thereof. The pharmaceutical composition has a synergistic effect against ovarian cancer.

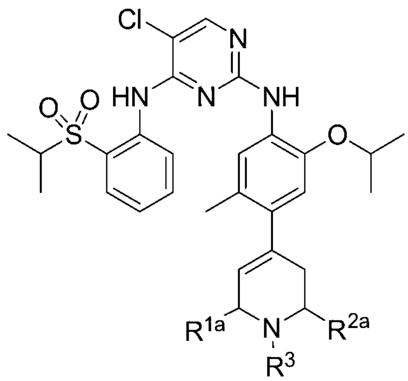

**(I)**

EP 4 684 788 A1

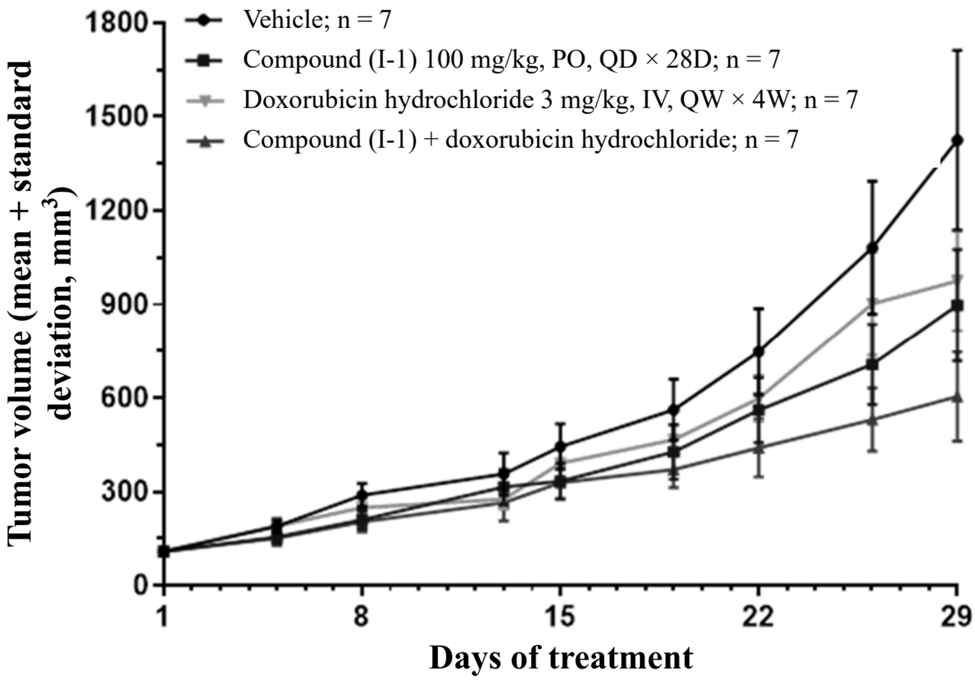
Vehicle; n = 7
Compound (I-1) 100 mg/kg, PO, QD × 28D; n = 7
Doxorubicin hydrochloride 3 mg/kg, IV, QW × 4W; n = 7
Compound (I-1) + doxorubicin hydrochloride; n = 7
Tumor volume (mean + standard deviation, mm3)
Days of treatment
0
300
600
900
1200
1500
1800
1
8
15
22
29

FIG. 1

**Description**

## TECHNICAL FIELD

The present disclosure relates to a pharmaceutical combination and use thereof.

## BACKGROUND

Ovarian cancer is the fifth leading common cause of cancer-related death among women. For patients with platinum-resistant ovarian cancer, the use of monotherapy, cytotoxic therapy, or hormonal therapy often yields a low response rate of about 10%. Therefore, improving the therapeutic efficacy for patients with platinum-resistant recurrent ovarian cancer is a pressing clinical problem to be addressed. Focal adhesion kinase (FAK) protein is highly expressed in about 68% of ovarian cancers, and FAK is considered an important antitumor target for malignant tumors, particularly ovarian cancer. To date, there are no approved small-molecule FAK inhibitors on the market. Five compounds have entered clinical trials as candidate anticancer drugs. The results of a phase 1b single-arm clinical trial evaluating the combination of the FAK inhibitor IN10018 and PLD in patients with platinum-resistant recurrent ovarian cancer showed that, as of December 31, 2021, the objective response rate (ORR) reached 56.7% and the disease control rate (DCR) was 86.7% among 30 evaluable patients (Wu et al., 2022), indicating that the combination of IN 10018 and PLD exerted a significant antitumor effect. The combination therapy has now entered the pivotal phase II study stage (CTR20221614).

In view of the lack of treatment methods for ovarian cancer in the prior art, to identify more effective treatment methods for ovarian cancer is an urgent technical challenge to be addressed.

## SUMMARY

The present disclosure is intended to address the technical problem of overcoming the lack of treatment methods for ovarian cancer in the prior art, and provides a novel pharmaceutical combination and use thereof. The pharmaceutical combination of the present disclosure exhibits a synergistic anti-ovarian cancer effect in a human ovarian cancer cell OVCAR3 mouse xenograft model.

The present disclosure addresses the above technical problem by the following methods.

The present disclosure provides a pharmaceutical combination, which comprises:

a substance X: a compound represented by formula (I), a pharmaceutically acceptable salt thereof, a solvate thereof, or a solvate of the pharmaceutically acceptable salt thereof; and

a substance Y: doxorubicin, a pharmaceutically acceptable salt thereof, a solvate thereof, or a solvate of the pharmaceutically acceptable salt thereof,

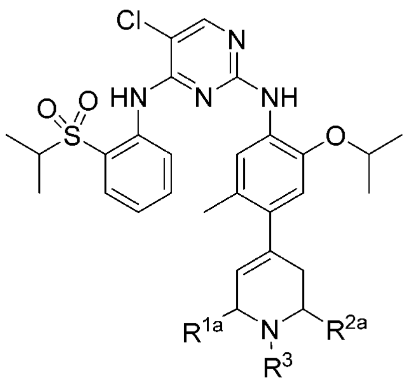

**(I)**

wherein in the compound represented by formula (I), $R^{1a}$ and $R^{2a}$ are each independently hydrogen, $C_{1-4}$ alkyl, or $C_{3-6}$ cycloalkyl; $R^3$ is

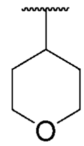
.

In one embodiment of the present disclosure, the pharmaceutical combination is a pharmaceutical combination for use in treating and/or preventing ovarian cancer.
In one embodiment of the present disclosure, the substance X is in a therapeutically effective amount.
In one embodiment of the present disclosure, the substance Y is in a therapeutically effective amount.
In one embodiment of the present disclosure, the substance X is the compound represented by formula (I) or the pharmaceutically acceptable salt thereof, for example, the compound represented by formula (I).
In one embodiment of the present disclosure, the substance Y is doxorubicin or the pharmaceutically acceptable salt thereof, for example, the pharmaceutically acceptable salt of doxorubicin.
In one embodiment of the present disclosure, the compound represented by formula (I) is a compound represented by formula (I-1):

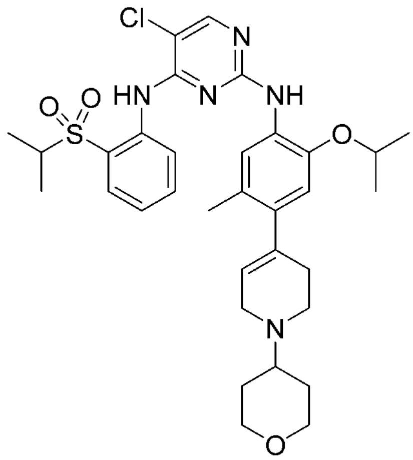

**(I-1)**

.

In one embodiment of the present disclosure, the substance X is the compound represented by formula (I-1).
In one embodiment of the present disclosure, the substance Y is doxorubicin hydrochloride.
In one embodiment of the present disclosure, the pharmaceutical combination comprises the substance X and the substance Y, the substance X is the compound represented by formula (I-1), and the substance Y is doxorubicin hydrochloride.
In one embodiment of the present disclosure, in the pharmaceutical combination, within one administration cycle, the mass ratio of the substance X to the substance Y is (200-1000):1, preferably (300-900):1, and more preferably (300-800):1.
In one embodiment of the present disclosure, in the pharmaceutical combination, the mass ratio of the substance X to the substance Y is (200-250):1, preferably 233:1.
In one embodiment of the present disclosure, the pharmaceutical combination comprises the substance X and the substance Y, the substance X is the compound represented by formula (I-1), and the substance Y is doxorubicin hydrochloride. The mass ratio of the substance X to the substance Y is (200-250):1, preferably 233:1.
In one embodiment of the present disclosure, the substance X is administered at a dose of 600 mg to 2000 mg, preferably 900 mg to 1500 mg, and more preferably 1200 mg, wherein the dose may be in the form of one dose or a plurality of doses.
In one embodiment of the present disclosure, the substance X is administered at a dose of 50 mg/kg to 200 mg/kg, for example, 80 mg/kg to 120 mg/kg, preferably 100 mg/kg, wherein the dose may be in the form of one dose or a plurality of doses.
In one embodiment of the present disclosure, the substance X is administered once daily.
In one embodiment of the present disclosure, the substance X is administered at a dose of 100 mg/kg once daily in one or a plurality of doses.
In one embodiment of the present disclosure, the substance X is administered by intragastric administration or oral administration, for example, intragastric administration.

In one embodiment of the present disclosure, the substance Y is administered by intravenous drip infusion at a dose of 40 $mg/m^2$, for example, 40 mg to 80 mg, such as 50 mg to 70 mg, wherein the dose may be in the form of one dose or a plurality of doses.

In one embodiment of the present disclosure, the substance Y is administered at a dose of 1 mg/kg to 15 mg/kg, for example, 2 mg/kg to 10 mg/kg, preferably 3 mg/kg, wherein the dose may be in the form of one dose or a plurality of doses.

In one embodiment of the present disclosure, the substance Y is administered once weekly.

In one embodiment of the present disclosure, the substance Y is administered once every 28 days.

In one embodiment of the present disclosure, the substance Y is administered at a dose of 3 mg/kg once weekly in one dose or a plurality of doses.

In one embodiment of the present disclosure, the substance Y is administered by intravenous injection, for example, by intravenous bolus injection.

In one embodiment of the present disclosure, the substance X is administered once daily, and the substance Y is administered once weekly in a three-week cycle.

In one embodiment of the present disclosure, the substance X is administered once daily at a dose of 100 mg/kg, and the substance Y is administered once weekly in a three-week cycle at a dose of 3 mg/kg.

In one embodiment of the present disclosure, the substance X is administered once daily at a dose of 600 mg to 2000 mg by intragastric administration or oral administration; the substance Y is administered at a dose of 40 mg to 80 mg once every 28 days, i.e., with each cycle being 28 days, by intravenous bolus injection.

In one embodiment of the present disclosure, the substance X is administered once daily at a dose of 100 mg/kg by intragastric administration or oral administration; the substance Y is administered once weekly in a three-week cycle at a dose of 3 mg/kg by intravenous bolus injection.

In one embodiment of the present disclosure, the substance X is the compound represented by formula (I-1), and the substance X is administered once daily at a dose of 100 mg/kg by intragastric administration or oral administration; the substance Y is doxorubicin hydrochloride, and the substance Y is administered once weekly in a three-week cycle at a dose of 3 mg/kg by intravenous bolus injection.

In one embodiment of the present disclosure, the substance X and the substance Y are used simultaneously, separately, or sequentially.

The present disclosure further provides a pharmaceutical composition A, which comprises:

a first pharmaceutical composition comprising a substance X and a pharmaceutically acceptable excipient, wherein the substance X is the compound represented by formula (I), the pharmaceutically acceptable salt thereof, the solvate thereof, or the solvate of the pharmaceutically acceptable salt thereof described above; and

a second pharmaceutical composition comprising a substance Y and a pharmaceutically acceptable excipient, wherein the substance Y is doxorubicin, a pharmaceutically acceptable salt thereof, a solvate thereof, or a solvate of the pharmaceutically acceptable salt thereof.

In one embodiment of the present disclosure, the pharmaceutical composition A is a pharmaceutical composition A for use in treating and/or preventing ovarian cancer.

In one embodiment of the present disclosure, in the first pharmaceutical composition, the substance X is in a therapeutically effective amount.

In one embodiment of the present disclosure, in the second pharmaceutical composition, the substance Y is in a therapeutically effective amount.

In one embodiment of the present disclosure, in the first pharmaceutical composition, the substance X is the compound represented by formula (I) or the pharmaceutically acceptable salt thereof, for example, the compound represented by formula (I).

Preferably, the compound represented by formula (I) is the compound represented by formula (I-1) described above.

In one embodiment of the present disclosure, in the second pharmaceutical composition, the substance Y is doxorubicin or the pharmaceutically acceptable salt thereof, for example, the pharmaceutically acceptable salt of doxorubicin.

In one embodiment of the present disclosure, in the first pharmaceutical composition, the substance X is the compound represented by formula (I-1) described above.

In one embodiment of the present disclosure, in the second pharmaceutical composition, the substance Y is doxorubicin hydrochloride.

In one embodiment of the present disclosure, the second pharmaceutical composition is a substance Y liposome, such as PLD, i.e., pegylated liposomal doxorubicin, also known as pegylated liposomal adriamycin. In the present disclosure, the PLD is a conventional PLD in the art, and preferably, the PLD is purchased from CSPC Ouyi Pharmaceutical Co., Ltd.

In one embodiment of the present disclosure, the pharmaceutical composition A comprises:

the first pharmaceutical composition comprising the substance X and the pharmaceutically acceptable excipient, the substance X being a compound represented by formula (I-1); and
a second pharmaceutical composition,
the second pharmaceutical composition being PLD.

In one embodiment of the present disclosure, the second pharmaceutical composition is the substance Y liposome, and in the pharmaceutical combination, within one administration cycle of 28 days, the mass ratio of the substance X to the substance Y liposome is (200-1000):1, preferably (300-900):1, and more preferably (300-800):1.

The mass ratio of the substance X to the substance Y liposome in the first pharmaceutical composition is (200-250):1, preferably 233:1.

In one embodiment of the present disclosure, the pharmaceutical composition A comprises:

the first pharmaceutical composition comprising the substance X and the pharmaceutically acceptable excipient, the substance X being a compound represented by formula (I-1); and
a second pharmaceutical composition,
the second pharmaceutical composition being PLD.

The mass ratio of the compound represented by formula (I-1) to the PLD is (200-1000):1, preferably (300-900):1, and more preferably (300-800):1.

In one embodiment of the present disclosure, in the first pharmaceutical composition, the substance X is administered at a dose of 50 mg/kg to 200 mg/kg, for example, 80 mg/kg to 120 mg/kg, preferably 100 mg/kg, wherein the dose may be in the form of one dose or a plurality of doses.

In one embodiment of the present disclosure, the substance X is administered at a dose of 600 mg to 2000 mg, preferably 900 mg to 1500 mg, and more preferably 1200 mg, wherein the dose may be in the form of one dose or a plurality of doses.

In one embodiment of the present disclosure, the first pharmaceutical composition is administered once daily.

In one embodiment of the present disclosure, in the first pharmaceutical composition, the substance X is administered at a dose of 100 mg/kg, and at this dose, the pharmaceutical composition may be administered once daily in one dose or a plurality of doses.

In one embodiment of the present disclosure, the first pharmaceutical composition is administered by intragastric administration or oral administration, for example, intragastric administration.

In one embodiment of the present disclosure, the second pharmaceutical composition is the substance Y liposome, and the substance Y liposome is administered at a dose of 1 mg/kg to 15 mg/kg, for example, 2 mg/kg to 10 mg/kg, preferably 3 mg/kg, wherein the dose may be in the form of one dose or a plurality of doses.

In one embodiment of the present disclosure, the substance Y is administered by intravenous drip infusion at a dose of 40 $mg/m^2$, for example, 40 mg to 80 mg, such as 50 mg to 70 mg, wherein the dose may be in the form of one dose or a plurality of doses.

In one embodiment of the present disclosure, the second pharmaceutical composition is administered once weekly or once every 28 days.

In one embodiment of the present disclosure, the second pharmaceutical composition is the substance Y liposome, the substance Y liposome is administered at a dose of 3 mg/kg, and at this dose, the pharmaceutical composition may be administered once weekly in one dose or a plurality of doses.

In one embodiment of the present disclosure, the second pharmaceutical composition is administered by intravenous injection, for example, by intravenous bolus injection.

In one embodiment of the present disclosure, the first pharmaceutical composition is administered once daily, and the second pharmaceutical composition is administered once weekly in a three-week cycle.

In one embodiment of the present disclosure, in the first pharmaceutical composition, the substance X is administered at a dose of 100 mg/kg, and the first pharmaceutical composition is administered once daily; the second pharmaceutical composition is the substance Y liposome, the substance Y liposome is administered at a dose of 3 mg/kg, and the second pharmaceutical composition is administered once weekly in a three-week cycle.

In one embodiment of the present disclosure, in the first pharmaceutical composition, the substance X is administered at a dose of 100 mg/kg, and the first pharmaceutical composition is administered once daily by intragastric administration or oral administration; the second pharmaceutical composition is the substance Y liposome, the substance Y liposome is administered at a dose of 3 mg/kg, and the second pharmaceutical composition is administered once weekly in a three-week cycle by intravenous bolus injection.

In one embodiment of the present disclosure, in the first pharmaceutical composition, the substance X is the compound represented by formula (I-1), the substance X is administered at a dose of 100 mg/kg, and the first pharmaceutical composition is administered once daily by intragastric administration or oral administration; the second pharmaceutical composition is pegylated liposomal doxorubicin, the pegylated liposomal doxorubicin is administered at a dose of 3 mg/kg, and the pegylated liposomal doxorubicin is administered once weekly in a three-week cycle by intravenous bolus injection.

In one embodiment of the present disclosure, the substance X is administered once daily at a dose of 600 mg to 2000 mg by intragastric administration or oral administration; the substance Y is administered at a dose of 40 mg to 80 mg once every 28 days, i.e., with each cycle being 28 days, by intravenous bolus injection.

In one embodiment of the present disclosure, the first pharmaceutical composition and the second pharmaceutical composition are administered simultaneously, separately, or sequentially.

The present disclosure further provides a combination kit, which comprises:

a first container comprising the first pharmaceutical composition according to any one of the embodiments described above; and

a second container comprising the second pharmaceutical composition according to any one of the embodiments described above.

The present disclosure further provides use of the pharmaceutical combination according to any one of the embodiments described above or the pharmaceutical composition A according to any one of the embodiments described above in preparing a medicament for preventing and/or treating ovarian cancer.

The present disclosure further provides a method for preventing and/or treating ovarian cancer, which comprises administering to a patient in need thereof the pharmaceutical combination according to any one of the embodiments described above or the pharmaceutical composition A according to any one of the embodiments described above.

The term "pharmaceutically acceptable salt" as used herein refers to a salt of the compound formed with relatively non-toxic and pharmaceutically acceptable acids or bases. When the compound contains relatively acidic functional groups, a base addition salt can be obtained by contacting the neutral form of such compound in a pure solution or suitable inert solvent with an adequate amount of a pharmaceutically acceptable base. When the compound contains relatively basic functional groups, an acid addition salt can be obtained by contacting the neutral form of such compound with an adequate amount of a pharmaceutically acceptable acid in a pure solution or a suitable inert solvent. Reference can be made to Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "treatment" or "treating" as used herein refers to therapeutic therapy. In the context of a specific condition, treatment refers to: (1) alleviating one or more biological manifestations of the disease or condition; (2) interfering with (a) one or more points in the biological cascade that causes or contributes to the condition or (b) one or more biological manifestations of the condition; (3) ameliorating one or more symptoms, effects, or side effects associated with the condition, or associated with the condition or the treatment thereof; or (4) slowing the progression of the condition or one or more biological manifestations of the condition.

The term "therapeutically effective amount" as used herein refers to an amount of the compound that, when administered to a subject, is sufficient to effectively treat the disease or condition described herein. The amount of compound constituting the "therapeutically effective amount" may vary depending on the compound, the condition and its severity, and the age of the subject to be treated, but may be adjusted as needed by those skilled in the art.

The term "container" as used herein refers to any container and closure suitable for storing, transporting, dispensing, and/or handling a pharmaceutical product.

The term "patient" as used herein refers to any animal, preferably a mammal, and most preferably a human, that is about to receive or has received administration of the compound or composition according to the embodiments of the present disclosure. The term "mammal" includes any mammal. Examples of mammals include, but are not limited to, cows, horses, sheep, pigs, cats, dogs, mice, rats, rabbits, guinea pigs, monkeys, humans, etc., with humans being the most preferred.

The term "pharmaceutically acceptable excipient" as used herein refers to excipients and additives used in the manufacture of a pharmaceutical product and in the formulation of a pharmaceutical formula, and refers to all substances, other than the active ingredient, contained in a pharmaceutical formulation. Reference can be made to Volume IV of the Pharmacopoeia of the People's Republic of China (2020 Edition), or Handbook of Pharmaceutical Excipients (Raymond C Rowe, 2009 Sixth Edition).

The above preferred conditions may be combined arbitrarily to obtain preferred embodiments of the present disclosure without departing from the general knowledge in the art.

The reagents and starting materials used in the present disclosure are commercially available.

The positive and progressive effect of the present disclosure is as follows: According to the present disclosure,

the compound represented by formula (I-1) is administered in combination with doxorubicin hydrochloride and pegylated liposomal doxorubicin, exhibiting a synergistic anti-ovarian cancer effect in a human ovarian cancer cell OVCAR3 mouse xenograft model.

## BRIEF DESCRIPTION OF THE DRAWINGS

FIG. 1 illustrates the antitumor effect of the compound represented by formula (I-1) administered in combination with doxorubicin hydrochloride in a human ovarian cancer OVCAR3 cell xenograft tumor model.
FIG. 2 illustrates the effect of the compound represented by formula (I-1) administered in combination with doxorubicin hydrochloride on body weight changes in a human ovarian cancer OVCAR3 cell xenograft tumor model.
FIG. 3 illustrates the antitumor effect of the compound represented by formula (I-1) administered in combination with PLD in a human ovarian cancer OVCAR3 cell xenograft tumor model.
FIG. 4 illustrates the effect of the compound represented by formula (I-1) administered in combination with PLD on body weight changes in a human ovarian cancer OVCAR3 cell xenograft tumor model.
FIG. 5 illustrates the effect of the compound represented by formula (I-1) administered in combination with doxorubicin hydrochloride on the growth of human ovarian cancer OVCAR3 cells.
FIG. 6 illustrates the effect of the compound represented by formula (I-1) administered in combination with doxorubicin hydrochloride on the growth of human ovarian cancer SK-OV-3 cells.
FIG. 7 illustrates the effect of the compound represented by formula (I-1) administered in combination with doxorubicin hydrochloride on the growth of human ovarian cancer A2780 cells.
FIG. 8 illustrates the effect of the compound represented by formula (I-1) administered in combination with doxorubicin hydrochloride on the growth of human ovarian cancer Kuramochi cells.
FIG. 9 illustrates the antitumor effect of the compound represented by formula (I-1) administered in combination with PLD in a murine ovarian cancer ID8 cell intraperitoneal model.
FIG. 10 illustrates the effect of the compound represented by formula (I-1) administered in combination with PLD on the survival time of mice in a murine ovarian cancer ID8 cell intraperitoneal model.
FIG. 11 illustrates the effect of the compound represented by formula (I-1) administered in combination with PLD on ascites formation in mice in a murine ovarian cancer ID8 cell intraperitoneal model.
FIG. 12 illustrates the effect of the compound represented by formula (I-1) administered in combination with PLD on mouse abdominal girth in a murine ovarian cancer ID8 cell intraperitoneal model.
FIG. 13 illustrates the effect of the compound represented by formula (I-1) administered in combination with PLD on the body weight of mice in a murine ovarian cancer ID8 cell intraperitoneal model.

## DETAILED DESCRIPTION

The present disclosure is further illustrated by the following examples, which are not intended to limit the present disclosure within the scope of the examples. Experimental methods without specified conditions in the following examples are conducted according to conventional methods and conditions, or according to product instructions.
The compound represented by formula (I-1) is as follows:

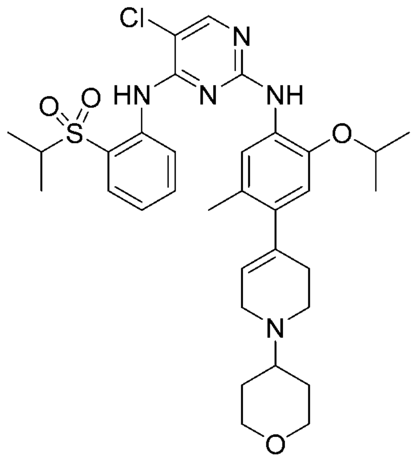

(I-1)

.

**Example 1**

**1. Experimental objective**

The antitumor effect of the compound represented by formula (I-1) in combination with doxorubicin hydrochloride or PLD in a human ovarian cancer cell OVCAR3 mouse xenograft model was evaluated.

**2. Experimental animals**

The animals used were female BALB/c Nude mice, aged 5 to 6 weeks. The body weight of the animals was (17-20) + 10% grams. The experimental animals were provided by Jiangsu GemPharmatech Co., Ltd., license number: SCXK (Su) 2018-0008. The animal certification numbers were: 202244524 (study No.: EF-13-2022) and 202272519 (study No.: EF-18-2022).

The experimental animals were all housed in the SPF-grade animal room at Suzhou Ascentage Pharma Co., Ltd. The daily care of the animals was performed by dedicated staff from the Experimental Animal Science Group of Suzhou Ascentage Pharma Co., Ltd., and the experimental studies were conducted by research personnel from Suzhou Ascentage Pharma Co., Ltd. The operation and management of all experimental animals strictly followed the Guidelines for the Use and Management of Experimental Animals of Suzhou Ascentage Pharma Co., Ltd.

The animals were group-housed in cages, with 6 to 7 mice per cage. The daily temperature was maintained at 20 °C to 26 °C, and the daily humidity was maintained at 40% to 70%, with a 12-hour light/dark cycle. The animals were continuously provided *ad libitum* access to a complete pelleted diet that had been sterilized by cobalt-60 irradiation. Drinking water was double-purified reverse osmosis water, sterilized by autoclaving before use. Water bottles were refilled continuously to allow free access. The padding was autoclaved wood shavings, which were replaced twice a week. The cage cards were labeled with information including the number of animals, sex, strain, study number, study initiation time, experiment personnel, source of animals, and group designation. Ear tags were used to mark the animals. The mice were acclimated for a minimum of 3 days before the experiment.

**3. Test substances**

**3.1 Compound represented by formula (I-1)**

The compound represented by formula (I-1) was provided by Jiangsu Ascentage Pharma Group Corp., Ltd. The compound represented by formula (I-1) was dissolved in a 20% PG/80% $NaH_2PO_4$ buffer, and the mixture was diluted to the final concentration according to the experimental protocol. The final solution was a clear solution. The compound represented by formula (I-1) was intragastrically administered at a dose of 100 mg/kg with a dosing volume of 10 mL/kg. The formulations for administration were prepared once every 3 days and stored at 4 °C when not in use. The preparation and use of the formulations for administration were performed under sterile conditions.

### 3.2 PLD (pegylated liposomal doxorubicin, also known as doxorubicin hydrochloride liposome)

The PLD was administered by intravenous injection at a dose of 3 mg/kg with a dosing volume of 10 mL/kg.

The doxorubicin hydrochloride liposome injection (batch No.: 691210421, specification: 10 mL:20 mg) purchased from CSPC Ouyi Pharmaceutical Co., Ltd. was diluted with 5% glucose solution to a concentration of 0.3 mg/mL and administered according to the administration regimen described above. The formulations for administration were freshly prepared before use and stored at 4 °C. The preparation and use of the formulations for administration were performed under sterile conditions.

### 3.3 Doxorubicin hydrochloride

Doxorubicin hydrochloride (batch No.: S120814, specification: 100 mg) was purchased from Selleck. Doxorubicin hydrochloride was administered by intravenous injection at a dose of 3 mg/kg with a dosing volume of 10 mL/kg. Doxorubicin hydrochloride was dissolved in normal saline to a concentration of 0.3 mg/mL. The formulations for administration were freshly prepared before use and stored at 4 °C. The preparation and use of the formulations for administration were performed under sterile conditions.

## 4. Other reagents

PG (propylene glycol) was purchased from SIGMA. $NaH_2PO_4$ was purchased from Sangon Biotech (Shanghai) Co., Ltd. Phosphoric acid was purchased from Sangon Biotech (Shanghai) Co., Ltd. 5% glucose was purchased from Hebei Kexing Pharmaceutical Co., Ltd. Normal saline was purchased from Sichuan Kelun Pharmaceutical Co., Ltd. The sterile syringes (1 mL) were purchased from Shanghai Kindly Enterprise Development Group (KDL) Co., Ltd. The stainless steel gavage needles were used after autoclaving.

Preparation of $NaH_2PO_4$ buffer: 1.56 g of $Na_2PO_4$ was weighed out, and the volume was adjusted to 1000 mL with deionized water. The pH was adjusted to 3 using phosphoric acid, and the buffer was autoclaved and then stored at room temperature for later use.

## 5. Cells

The human ovarian cancer OVCAR3 cells were purchased from China Center for Type Culture Collection (CCTCC). The culture conditions were as follows: RPMI-1640 (containing 10 mM HEPES buffer and 1 mM sodium pyruvate) supplemented with 20% fetal bovine serum and 1% penicillin and streptomycin. RPMI-1640 (Shanghai Yishan Biotechnology Co., Ltd., Cat. ES-RG001), FBS (SIGMA, Cat. F8318), Penicillin-Streptomycin (gibco, Cat. 15140-122), HEPES buffer (Shanghai BasalMedia Technologies Co., Ltd., Cat. B110JV), sodium pyruvate (gibco, Cat. 11360-070), PBS (Keyoubo Biotechnology Co., Ltd., Cat. U10017B), Trypsin (gibco, Cat. 25200-072), and Matrigel (Coming, Cat. 354234). The cells were cultured in an incubator at 37 °C with 5% $CO_2$.

## 6. Instruments

Biosafety cabinet (model: AC2-6S1, ESCO); carbon dioxide cell incubator (model: CLM-170B-8-CF, ESCO); inverted microscope (model: CKX53, Olympus); balance (model: XSR205DU, Mettler Toledo); low-speed centrifuge (model: L600, Shanghai Luxiangyi Centrifuge Instrument Co., Ltd.); constant-temperature water bath kettle (model: DK-8AX, Shanghai Yiheng Scientific Instruments Co., Ltd.); automated cell counter (model: JSY-SC-021H, Guangzhou BodBoge Technology Co., Ltd.); and digital vernier caliper (model: 16EWRI4103403, Mahr GmbH).

## 7. Experimental design

### 7.1 Experimental design 1

A total of 71 immunodeficient mice were subcutaneously injected with $10 \times 10^6$ OVCAR3 cells to establish a xenograft tumor model. Tumor-bearing mice with uniform tumors were randomly grouped into different treatment groups based on tumor volume, with 7 mice in each group. The experimental design is shown in Table 1.

Tumor cells: OVCAR3; $10 \times 10^6$ cells + Matrigel gel/mouse. A total of 71 mice were inoculated. 28 tumor-bearing mice with relatively uniform tumors were selected and randomly divided into 4 experimental groups, with 7 mice per group.

Table 1. Experimental design 1

| Group | Number of animals | Drug used | Route of administration | Dose | Frequency and time of administration |
|---|---|---|---|---|---|
| 1 | 7 | Vehicle control group | p.o. | - | qd × 28d |
| 2 | 7 | Compound represented by formula (I-1) | p.o. | 100 mg /kg | qd × 28d |
| 3 | 7 | Doxorubicin hydrochloride | i.v. | 3 mg/kg | qw × 4w |
| 4 | 7 | Compound represented by formula (I-1) + doxorubicin hydrochloride | p.o.<br>i.v. | 100 mg/kg<br>3 mg/kg | qd × 28d<br>qw × 4w |

**7.2 Experimental design 2**

A total of 56 immunodeficient mice were subcutaneously injected with 10 × $10^6$ OVCAR3 cells to establish a xenograft tumor model. Tumor-bearing mice with uniform tumors were randomly grouped into different treatment groups based on tumor volume, with 6 mice in each group. The experimental design is shown in Table 2.

Tumor cells: OVCAR3; 10 × $10^6$ cells + Matrigel gel/mouse. A total of 56 mice were inoculated. 24 tumor-bearing mice with relatively uniform tumors were selected and randomly divided into 4 experimental groups, with 6 mice in each group.

Table 2. Experimental design 2

| Group | Number of animals | Drug used | Route of administration | Dose | Frequency and time of administration |
|---|---|---|---|---|---|
| 1 | 6 | Vehicle control group | p.o. | - | qd × 21d |
| 2 | 6 | Compound represented by formula (I-1) | p.o. | 100 mg /kg | qd × 21d |
| 3 | 6 | PLD | i.v. | 3 mg/kg | qw × 3w |
| 4 | 6 | Compound represented by formula (I-1) + PLD | p.o.<br>i.v. | 100 mg/kg<br>3 mg/kg | qd × 21d<br>qw × 3w |

**7.3 Experimental method**

Under sterile conditions, a xenograft tumor model was established by subcutaneous injection of tumor cells into the right dorsal side of immunodeficient mice. When the tumor reached an appropriate size (100 $mm^3$ to 200 $mm^3$), the animals were randomly grouped by randomized block method based on the tumor volume of the animals, the tumor volume difference of each group should be less than 20% of the mean value, with 6 to 7 animals in each group, and administration was started on the day of grouping (i.e., d1). The body weight and tumor size of the animals were measured twice a week during the experiment. The clinical symptoms were observed and recorded daily. At the end of administration or at the end of the experiment.

The tumor-related parameters were calculated with reference to the Technical Guidelines for Non-Clinical Studies of Cytotoxic Antitumor Drugs (2006) issued by China Food and Drug Administration (CFDA).

The tumor volume (TV) was calculated as follows: TV = a × $b^2$/2, where a and b represent the measured length and width of the tumor, respectively.

The relative tumor volume (RTV) was calculated as follows: RTV = $V_t/V_I$, where $V_I$ represents the tumor volume at the time of grouping and administration (day 1), and $V_t$ represents the tumor volume at the time of measurement.

The evaluation parameter of antitumor activity was relative tumor proliferation rate T/C (%), and the calculation formula was as follows: tumor proliferation rate T/C (%) = ($T_{RTV}/C_{RTV}$) × 100%, where $T_{RTV}$ represents the RTV of the treatment group, and $C_{RTV}$ represents the RTV of the negative control group.

The change of body weight (%) of the animals = (measured body weight - body weight at the time of grouping)/body weight at the time of grouping $\times$ 100%.

The synergy score was calculated using the following formula (Clarke R, 1997):

$$\text{synergy score} = ((A/C) \times (B/C))/(AB/C),$$

where A represents response to drug A; B represents response to drug B; C represents response to vehicle control; AB represents response to combination treatment with A and B.

Evaluation criteria for efficacy: According to the Technical Guidelines for Non-Clinical Studies of Cytotoxic Antitumor Drugs (November 2006) issued by CFDA, the compound was considered effective when T/C (%) was $\leq$ 40% with $p < 0.05$ by statistical analysis for RTV. If the body weight of the mice decreased by more than 20% or the drug-related deaths exceeded 20%, the dose of the drug was considered to have severe toxicity. The animals were euthanized according to animal welfare principles when the body weight loss of the animals exceeded 20%, disease progression or moribund state was observed, or the tumor burden exceeded 10% of the animal's body weight.

### 7.4 Data analysis

The antitumor growth curves of the test substances were plotted by taking the X-axis as the treatment time (day) and the corresponding tumor volume (mean value) as the Y-axis. One-way ANOVA was used to compare intergroup differences in tumor volume, and Games-Howell test was employed for intergroup comparison when a significant difference in the F value (a ratio of treatment variance to the error variance) was observed. All data were statistically analyzed using SPSS (Statistical Product and Service Solutions) software (version 18.0, IBM, Armonk, NY, U.S.). Graphing was performed using Prism version 6 (GraphPad Software Inc., San Diego, CA).

For combination treatment, a synergy score of <1 indicated antagonistic effect, a synergy score of =1 indicated additive effect, and a synergy score of > 1 indicated synergistic effect.

### 7.5 Results

#### 7.5.1 Antitumor effect of compound represented by formula (I-1) administered in combination with doxorubicin hydrochloride in OVCAR3 model

In this experiment, the combined therapeutic effect of the compound represented by formula (I-1) and doxorubicin hydrochloride was evaluated in an OVCAR3 xenograft tumor model.

The results showed that: as shown in Table 3 and FIG. 1, the compound represented by formula (I-1) monotherapy group and the doxorubicin hydrochloride monotherapy group exhibited moderate tumor growth inhibition. The compound represented by formula (I-1) was administered at a dose of 100 mg/kg, p.o., q.d., for 28 days, and the T/C value of the compound represented by formula (I-1) treatment group on day 29 was 63.15%. Doxorubicin hydrochloride was administered at a dose of 3 mg/kg, i.v., q.w., for 4 weeks, and the T/C value of the doxorubicin hydrochloride monotherapy group on day 29 was 70.50%. The T/C value of the combination treatment with the compound represented by formula (I-1) and doxorubicin hydrochloride was 42.32% ($P < 0.01$ compared with the vehicle control group), indicating that the combination treatment with the two drugs exhibited a certain inhibitory effect on tumor growth. The synergy score of the combination treatment group was 1.05, suggesting a synergistic effect. During the treatment, no significant body weight loss was observed in the animals of each group, and the animals were in good condition (Table 3 and FIG. 2). Conclusion: In the human ovarian adenocarcinoma OVCAR3 xenograft tumor model, the antitumor effect of the compound represented by formula (I-1) administered in combination with doxorubicin hydrochloride was superior to that of the compound represented by formula (I-1) or doxorubicin hydrochloride used alone.

Table 3. Effect of combination treatment with compound represented by formula (I-1) and doxorubicin hydrochloride

| Treatment | RTV on day 29 after administration (mean value ± standard error) | T/C (%) on day 29 after administration | Body weight change (%) on day 29 after administration | Synergy score[a] on day 29 after administration |
|---|---|---|---|---|
| Vehicle control | 12.59 + 2.07 | - | 10.94 | - |
| Compound represented by formula (I-1) 100 mg/kg | 7.95 ± 1.31 | 63.15 | -3.01 | - |

(continued)

| Treatment | RTV on day 29 after administration (mean value ± standard error) | T/C (%) on day 29 after administration | Body weight change (%) on day 29 after administration | Synergy score[a] on day 29 after administration |
|---|---|---|---|---|
| Doxorubicin hydrochloride 3 mg/kg | 8.88 + 1.19 | 70.50 | 6.94 | - |
| Compound represented by formula (I-1) + doxorubicin hydrochloride | 5.33 ± 1.10** | 42.32 | -2.47 | 1.05 |
| | ** represents P < 0.01 compared with the vehicle group. For [a]synergy score, the value > 1 represents synergistic effect, the value = 1 represents additive effect, and the value < 1 represents antagonistic effect. | | | |

**7.5.2 Antitumor effect of compound represented by formula (I-1) administered in combination with PLD in OVCAR3 model**

Clinically, the incidence of cardiotoxicity induced by PLD is significantly lower than that induced by doxorubicin. In this experiment, the combined therapeutic effect of the compound represented by formula (I-1) and PLD was further evaluated in an OVCAR3 xenograft tumor model.

The results showed that: as shown in Table 4 and FIG. 3, the compound represented by formula (I-1) monotherapy group and the doxorubicin hydrochloride monotherapy group exhibited moderate tumor growth inhibition. The compound represented by formula (I-1) was administered at a dose of 100 mg/kg, p.o., q.d., for 21 days, and the T/C value of the compound represented by formula (I-1) monotherapy group on day 22 was 72.77%. The PLD was administered at a dose of 3 mg/kg, i.v., q.w., for 3 weeks, and the T/C value of the PLD monotherapy group on day 22 was 57.36%. The T/C value of the combination treatment with the compound represented by formula (I-1) and PLD was 31.49% (P < 0.001 compared with the vehicle control group; P < 0.05 compared with the monotherapy group of the compound represented by formula (I-1)), indicating that the combination treatment with the two drugs exhibited a significantly increased inhibitory effect on tumor growth. The synergy score of the combination treatment group was 1.33, exhibiting a synergistic effect. During the treatment, no significant body weight loss was observed in the animals of each group, and the animals were in good condition (Table 4 and FIG. 4).

Conclusion: In the human ovarian adenocarcinoma OVCAR3 xenograft tumor model, the antitumor effect of the compound represented by formula (I-1) administered in combination with PLD was significantly superior to that of the compound represented by formula (I-1) or PLD used alone.

Table 4. Effect of combination treatment with compound represented by formula (I-1) and PLD

| Treatment | RTV on day 22 after administration (mean value + standard error) | T/C (%) on day 22 after administration | Body weight change (%) on day 22 after administration | Synergy score[a] on day 22 after administration |
|---|---|---|---|---|
| Vehicle control | 10.20 + 0.99 | - | 13.26 | - |
| Compound represented by formula (I-1) 100 mg/kg | 7.42 ± 1.06 | 72.77 | 5.55 | - |
| PLD 3 mg/kg | 5.85 + 0.80** | 57.36 | 9.17 | - |
| Compound represented by formula (I-1) + PLD | 3.21 ± 0.47***# | 31.49 | 1.40 | 1.33 |

(continued)

| Treatment | RTV on day 22 after administration (mean value + standard error) | T/C (%) on day 22 after administration | Body weight change (%) on day 22 after administration | Synergy score[a] on day 22 after administration |
|---|---|---|---|---|
| | ** represents $P < 0.01$ compared with the vehicle group; *** represents $P < 0.001$ compared with the vehicle group; # represents $P < 0.05$ compared with the compound represented by formula (I-1) group. For [a]synergy score, the value > 1 represents synergistic effect, the value = 1 represents additive effect, and the value < 1 represents antagonistic effect. | | | |

**Example 2.**

**1. Experimental objective**

The effect of the compound represented by formula (I-1) in combination with doxorubicin hydrochloride on inhibiting the growth of human ovarian cancer cells OVCAR3, SK-OV-3, A2780, and Kuramochi was evaluated.

**2. Test substances**

**2.1 Compound represented by formula (I-1)**

The compound represented by formula (I-1) was provided by Jiangsu Ascentage Pharma Group Corp., Ltd. The compound represented by formula (I-1) was dissolved in DMSO to prepare a stock solution at a concentration of 10 mM, and the solution was diluted to the final concentration according to the experimental protocol. The final solution was a clear solution. The preparation and use of the solution were performed under sterile conditions.

**2.3 Doxorubicin hydrochloride**

Doxorubicin hydrochloride (batch No.: S120814, specification: 100 mg) was purchased from Selleck. The compound was dissolved in DMSO to prepare a stock solution at a concentration of 10 mM, and the solution was diluted to the final concentration according to the experimental protocol. The final solution was a clear solution. The preparation and use of the solution were performed under sterile conditions.

**3. Cells**

The human ovarian cancer OVCAR3 cells were purchased from China Center for Type Culture Collection (CCTCC). Human ovarian cancer cells A2780, Kuramochi, and SK-OV-3 were purchased from Nanjing Cobioer Biosciences Co., Ltd. The culture conditions of OVCAR3 were as follows: RPMI-1640 (containing 10 mM HEPES buffer and 1 mM sodium pyruvate) supplemented with 20% fetal bovine serum and 1% penicillin and streptomycin. The culture conditions of A2780 and Kuramochi were as follows: RPMI-1640 (containing 10 mM HEPES buffer and 1 mM sodium pyruvate) supplemented with 10% fetal bovine serum and 1% penicillin and streptomycin. The culture conditions of SK-OV-3 were as follows: McCoy'5A (containing 10 mM HEPES buffer and 1 mM sodium pyruvate) supplemented with 10% fetal bovine serum and 1% penicillin and streptomycin. RPMI-1640 (Shanghai Yishan Biotechnology Co., Ltd., Cat. ES-RG001), McCoy'5A (gibco, Cat. 16600-082), FBS (SIGMA, Cat. F8318), Penicillin-Streptomycin (gibco, Cat. 15140-122), HEPES buffer (Shanghai BasalMedia Technologies Co., Ltd., Cat. B110JV), sodium pyruvate (gibco, Cat. 11360-070), PBS (Keyoubo Biotechnology Co., Ltd., Cat. U10017B), and Trypsin (gibco, Cat. 25200-072). The cells were cultured in an incubator at 37 °C with 5% $CO_2$.

**4. Instruments**

Biosafety cabinet (model: AC2-6S1, ESCO); carbon dioxide cell incubator (model: CLM-170B-8-CF, ESCO); inverted microscope (model: CKX53, Olympus); balance (model: XSR205DU, Mettler Toledo); low-speed centrifuge (model: L600, Shanghai Luxiangyi Centrifuge Instrument Co., Ltd.); constant-temperature water bath kettle (model: DK-8AX, Shanghai Yiheng Scientific Instruments Co., Ltd.); automated cell counter (model: JSY-SC-021H, Guangzhou BodBoge Technology Co., Ltd.); and microplate reader (SpectraMax Plus 384, Molecular Devices, LLC., US).

### 5. Experimental design

Cell seeding: The antiproliferative effects of the compounds were evaluated by cell titer glo (CellTiter Glo Kit, Promega) assays. The cells were seeded into a 96-well plate. For each negative control group, only 50 μL of complete medium was added. For each test well, 50 μL of complete medium cell suspension was added. The cell density was (5-10) × 10^4 cells/well. Compound treatment (in the dark): In the 96-well culture plate, an appropriate maximum concentration was selected based on the sensitivity of different cells to different drugs, and serial dilution was performed in a ratio of 1:2 or 1:3 to obtain 6 or 9 concentrations. 50 μL of compound-containing medium was added to each well, and 2 to 3 replicate wells were made for each concentration. After compound addition, the 96-well plate was incubated in an incubator at 37 °C with 5% $CO^2$. The combination effect of the compound represented by formula (I-1) and doxorubicin hydrochloride was evaluated by treating the cells for 72 hours with various concentrations of doxorubicin hydrochloride in combination with 3 fixed concentrations of the compound represented by formula (I-1).

Reading and data analysis: At the end of the culture, the reaction substrate in the CellTiter Glo Kit was added, and the chemiluminescence values were measured using a microplate reader. The mean OD value of the duplicate wells was used to calculate the percentage of cell viability according to the following formula: (test well - blank control well)/(cell control well- blank control well) × 100%. The $IC_{50}$ value was calculated using the nonlinear regression data analysis method of Graphpad Prism9. The results are shown in FIGs. 5 to 8. For the combination experiment, the cell viability was calculated after normalization treatment of the mean OD value of the 3 replicate wells of the single-drug control group.

The results are shown in FIGs. 5 to 8. By comparing the $IC_{50}$ values obtained from the curves of combination treatment and single-drug treatment, it was shown that the 2 compounds achieved synergistic effect (the curve of combination treatment was shifted to the left).

### Example 3

### 1. Experimental objective

The antitumor effect of the compound represented by formula (I-1) in combination with PLD in a mouse ovarian cancer cell ID-8 intraperitoneal metastasis model was evaluated.

### 2. Experimental animals

The animals used were female C57BL/6 mice, aged 6 to 8 weeks. The experimental animals were provided by Zhejiang Vital River Laboratory Animal Technology Co., Ltd. (license number: SCXK (Zhe) 2019-0001). The animal certificate number was: 20230628Abzz0619000108.

The experimental animals were all housed in the SPF-grade animal room at Suzhou Ascentage Pharma Co., Ltd. The daily care of the animals was performed by dedicated staff from the Experimental Animal Science Group of Suzhou Ascentage Pharma Co., Ltd., and the experimental studies were conducted by research personnel from Suzhou Ascentage Pharma Co., Ltd. The operation and management of all experimental animals strictly followed the Guidelines for the Use and Management of Experimental Animals of Suzhou Ascentage Pharma Co., Ltd.

### 3. Test substances

#### 3.1 Compound represented by formula (I-1)

The compound represented by formula (I-1) was provided by Jiangsu Ascentage Pharma Group Corp., Ltd. The compound represented by formula (I-1) was dissolved in a 20% PG/80% $NaH_2PO_4$ buffer, and the mixture was diluted to the final concentration according to the experimental protocol. The final solution was a clear solution. The compound represented by formula (I-1) was intragastrically administered at a dose of 100 mg/kg. The formulations for administration were prepared once every 3 days and stored at 4 °C when not in use. The preparation and use of the formulations for administration were performed under sterile conditions.

#### 3.2 PLD (pegylated liposomal doxorubicin, also known as doxorubicin hydrochloride liposome)

The PLD was administered by intravenous injection at a dose of 3 mg/kg. The doxorubicin hydrochloride liposome injection (batch No.: 691210421, specification: 10 mL:20 mg) purchased from CSPC Ouyi Pharmaceutical Co., Ltd. was diluted with 5% glucose solution to a concentration of 0.3 mg/mL and administered according to the administration regimen described above. The formulations for administration were freshly prepared before use and stored at 4 °C. The preparation and use of the formulations for administration were performed under sterile conditions.

**4. Other reagents**

PG (propylene glycol) was purchased from SIGMA. $NaH_2PO_4$ was purchased from Sangon Biotech (Shanghai) Co., Ltd. Phosphoric acid was purchased from Sangon Biotech (Shanghai) Co., Ltd. 5% glucose was purchased from Hebei Kexing Pharmaceutical Co., Ltd. Normal saline was purchased from Sichuan Kelun Pharmaceutical Co., Ltd. The sterile syringes (1 mL) were purchased from Shanghai Kindly Enterprise Development Group (KDL) Co., Ltd. The stainless steel gavage needles were used after autoclaving. Preparation of $NaH_2PO_4$ buffer: 1.56 g of $Na_2PO_4$ was weighed out, and the volume was adjusted to 1000 mL with deionized water. The pH was adjusted to 3 using phosphoric acid, and the buffer was autoclaved and then stored at room temperature for later use. D-Luciferin Potassium Salt Bioluminescent Substrate (luciferin potassium salt) was purchased from PerkinElmer.

**5. Cells**

Mouse ovarian cancer ID8 cells were purchased from Nanjing Cobioer, and the cells stably expressed luciferase, enabling its use for *in vivo* imaging in small animals. The culture conditions were as follows: RPMI-1640 (containing 10 mM HEPES buffer and 1 mM sodium pyruvate) supplemented with 10% fetal bovine serum and 1% penicillin and streptomycin. RPMI-1640 (Shanghai Yishan Biotechnology Co., Ltd., Cat. ES-RG001), FBS (SIGMA, Cat. F8318), Penicillin-Streptomycin (gibco, Cat. 15140-122), HEPES buffer (Shanghai BasalMedia Technologies Co., Ltd., Cat. B110JV), PBS (Keyoubo Biotechnology Co., Ltd., Cat. U10017B), Trypsin (gibco, Cat. 25200-072), and Matrigel (Coming, Cat. 354234). The cells were cultured in an incubator at 37 °C with 5% $CO_2$.

**6. Instruments**

Biosafety cabinet (model: AC2-6S1, ESCO); carbon dioxide cell incubator (model: CLM-170B-8-CF, ESCO); inverted microscope (model: CKX53, Olympus); balance (model: XSR205DU, Mettler Toledo); low-speed centrifuge (model: L600, Shanghai Luxiangyi Centrifuge Instrument Co., Ltd.); constant-temperature water bath kettle (model: DK-8AX, Shanghai Yiheng Scientific Instruments Co., Ltd.); automated cell counter (model: JSY-SC-021H, Guangzhou BodBoge Technology Co., Ltd.); and IVIS® Spectrum In Vivo Imaging System (model: Lumina 3, PerkinElmer).

**7. Experimental design**

**7.1 Experimental design**

A total of 90 C57BL/6 mice were intraperitoneally injected with $15 \times 10^6$ ID8 cells to establish an ovarian cancer intraperitoneal model. Tumor-bearing mice with uniform tumors were randomly grouped into different treatment groups based on *in vivo* imaging data, with 6 to 7 mice in each group. The experimental design is shown in Table 5.

Table 5. Experimental design 1

| Group | Number of animals | Drug used | Route of administration | Dose | Frequency and time of administration |
|---|---|---|---|---|---|
| 1 | 7 | Vehicle control group | p.o./i.v. | - | qd × 21d |
| 2 | 7 | Compound represented by formula (I-1) | p.o. | 100 mg /kg | qd × 21d |
| 3 | 7 | PLD | i.v. | 3 mg/kg | qw × 3w |
| 4 | 7 | Compound represented by formula (I-1) + PLD | p.o.<br>i.v. | 100 mg/kg<br>3 mg/kg | qd × 21d<br>qw × 3w |

**7.2 Experimental method**

Two weeks after intraperitoneal inoculation, the mice were randomly grouped by randomized block method based on the tumor volume value (Total flux) calculated through *in vivo* imaging. The tumor volume difference of each group should be less than 20% of the mean value, with 6 to 7 animals in each group, and administration was started on the day of grouping (i.e., d1). The body weight and tumor volume of the animals were measured twice or thrice a week during the experiment. The clinical symptoms were observed and recorded daily. After the administration was completed, the

abdominal girth, body weight, and survival time of the mice were continuously observed until all the animals were subjected to euthanasia.

The tumor volume (TV) was measured using an IVIS imaging system, and the total luminescence value (Total flux) of each mouse was calculated under the same measurement area.

The relative tumor volume (RTV) was calculated as follows: RTV = $V_t/V_I$, where $V_I$ represents the tumor volume at the time of grouping and administration (day 1), and $V_t$ represents the tumor volume at the time of measurement.

The evaluation parameter of antitumor activity was relative tumor proliferation rate T/C (%), and the calculation formula was as follows: tumor proliferation rate T/C (%) = ($T_{RTV}/C_{RTV}$) × 100%, where $T_{RTV}$ represents the RTV of the treatment group, and $C_{RTV}$ represents the RTV of the negative control group.

The change of body weight (%) of the animals = (measured body weight - body weight at the time of grouping)/body weight at the time of grouping × 100%.

The synergy score was calculated using the following formula (Clarke R, 1997):

$$\text{synergy score} = ((A/C) \times (B/C))/(AB/C),$$

where A represents response to drug A; B represents response to drug B; C represents response to vehicle control; AB represents response to combination treatment with A and B.

The evaluation criteria of efficacy followed the Technical Guidelines for Non-Clinical Studies of Cytotoxic Antitumor Drugs (November 2006) issued by CFDA. The animals were euthanized according to animal welfare principles when the body weight loss of the animals exceeded 20%, disease progression or moribund state was observed, the tumor burden exceeded 10% of the animal's body weight, the abdominal girth exceeded 100 mm, or the body weight exceeded 30 g.

### 7.3 Data analysis

The antitumor growth curves of the test substances, the survival time, and ascites production time of the mice were plotted by taking the X-axis as the treatment time (day), and the corresponding tumor volume (mean value), the survival rate, and the ascites-free rate as the Y-axis. One-way ANOVA was used to compare intergroup differences in tumor volume, and Games-Howell test was employed for intergroup comparison when a significant difference in the F value (a ratio of treatment variance to the error variance) was observed. The data were statistically analyzed using SPSS (Statistical Product and Service Solutions) software (version 18.0, IBM, Armonk, NY, U.S.). Graphing was performed using Prism version 9 (GraphPad Software Inc., San Diego, CA), and the survival time and ascites production time were analyzed using the log-rank test. For combination treatment, a synergy score of <1 indicated antagonistic effect, a synergy score of =1 indicated additive effect, and a synergy score of >1 indicated synergistic effect.

### 7.4 Results

In this experiment, the combined therapeutic effect of the compound represented by formula (I-1) and PLD was evaluated in an ID8 model. The compound represented by formula (I-1) was administered at a dose of 100 mg/kg, p.o., q.d., for 21 days, and PLD was administered at a dose of 3 mg/kg, i.v., q.w., for 3 weeks.

The results showed that: the tumor volume of the mice was measured on day 22, and the results are shown in FIG. 9; the T/C of the compound represented by formula (I-1) monotherapy group, the PLD monotherapy group, and the two-drug combination treatment group were 37.49%, 16.93%, and 6.65%, respectively. As shown in Table 6, the T/C value of the compound represented by formula (I-1) monotherapy group on day 36 was 97.13%. The T/C value of the PLD monotherapy group on day 36 was 33.39%. The T/C value of the combination treatment group with the compound represented by formula (I-1) and PLD on day 36 was 15.13%, and the synergy score of the combination treatment group was up to 2.14, indicating that the combination of the two drugs exhibited a strong inhibitory effect on tumor growth. Since the imaging accuracy would be affected by ascites produced in the later stage of the model, the abdominal girth, ascites progression, and survival time of the mice were measured to replace the *in vivo* imaging to evaluate the efficacy. As shown in FIG. 10, compared with the solvent control group, the survival time of the mice in the compound represented by formula (I-1) and PLD treatment groups was prolonged, and the survival time of the mice in the two-drug combination treatment group was significantly prolonged compared with that in the solvent control group and that in the monotherapy groups, demonstrating that the combination therapy exhibited a synergistic effect in prolonging the survival time of the mice. As shown in FIGs. 11 and 12, the development and progression of ovarian cancer in the mice were characterized by detecting the production of ascites and measuring the abdominal girth of the mice, and it was found that the combination treatment with the two drugs could significantly prolong the progression-free survival time of the mice as compared to monotherapy. As shown in FIG. 13, during the treatment, no significant body weight loss was observed in the animals of each group, and

the animals were in good condition.

Conclusion: In the ID8 model, the antitumor effect of the compound represented by formula (I-1) administered in combination with PLD was superior to that of the compound represented by formula (I-1) or PLD used alone.

Table 6. Effect of combination treatment with compound represented by formula (I-1) and PLD

| **Treatment** | **RTV on day 36 after administration (mean value ± standard error)** | **T/C (%) on day 36 after administration** | **Synergy score on day 36 after administration** |
|---|---|---|---|
| Vehicle control | 4.86 + 0.63 | - | - |
| Compound represented by formula (I-1) 100 mg/kg | 4.72 ± 1.35 | 97.13 | - |
| PLD 3 mg/kg | 1.62 ± 0.61* | 33.39 | - |
| Compound represented by formula (I-1) (100 mg/kg) + PLD (3 mg/kg) | 0.73 ± 0.14**†† | 15.13 | 2.14 |
| * represents p < 0.05, ** represents p < 0.01, *** represents p < 0.001, vs. vehicle; † represents p < 0.05, †† represents p < 0.01, ††† represents p < 0.001, vs. APG-2449. | | | |

**Example 4**

Title: treatment of patients with platinum-resistant recurrent ovarian cancer or advanced solid tumors using APG-2449 alone or in combination with PLD.

Objective: The study was an open, multicenter, and dose-finding phase I clinical trial aimed to assess the safety of APG-2449 monotherapy in the treatment of patients with advanced solid tumors, and to assess the safety, tolerability, and efficacy of APG-2449 in combination with PLD in the treatment of ovarian cancer.

Study design:

The study included two parts:

Part A was: APG-2449 monotherapy for the treatment of advanced solid tumors.
APG-2449 was administered orally once daily (QD) after meals. The recommended Phase II dose (RP2D) for monotherapy was 1200 mg, and continuously administered in 28-day treatment cycles, to evaluate its safety and pharmacokinetic characteristics.
Part B was: dose investigation and expansion of APG-2449 in combination with PLD.

Drug: APG-2449 was administered once daily (QD) at a dose of 1200 mg each time, with every 28 days as an administration cycle.

Drug: PLD was administered at a dose of 40 $mg/m^2$ by intravenous drip infusion on the first day of each 28-day cycle.

A standard "3+3" design was employed. Subsequent treatment groups may receive an increased or decreased dose of APG-2449 at 1500 mg or 900 mg, respectively. Two doses would be selected for expansion to evaluate the efficacy of the combination treatment.

Endpoints:

1. Treatment-related adverse events in NCI-CTCAE version 5.0.

The number and frequency of adverse events of the test drug were assessed according to CTCAE v5.0. The number of patients with adverse events, and the number of patients with abnormal vital signs, abnormal physical examination, abnormal laboratory findings, and abnormal 12-lead electrocardiography in the APG-2449 monotherapy group and the combination treatment group with PLD were assessed.

2. Dose-limiting toxicity (DLT).

DLT was defined according to the incidence of drug-related grade 3-5 adverse events occurring within the first 4 weeks of study treatment. These would be evaluated according to NCI-CTCAE version 5.0.

Although specific embodiments of the present disclosure have been described above, it will be understood by those skilled in the art that these embodiments are merely illustrative and that many changes or modifications can be made to these embodiments without departing from the principle and spirit of the present disclosure. Therefore, the protection scope of the present disclosure is defined by the appended claims.

## Claims

**1.** A pharmaceutical combination, comprising:

a substance X: a compound represented by formula (I), a pharmaceutically acceptable salt thereof, a solvate thereof, or a solvate of the pharmaceutically acceptable salt thereof; and
a substance Y: doxorubicin, a pharmaceutically acceptable salt thereof, a solvate thereof, or a solvate of the pharmaceutically acceptable salt thereof,

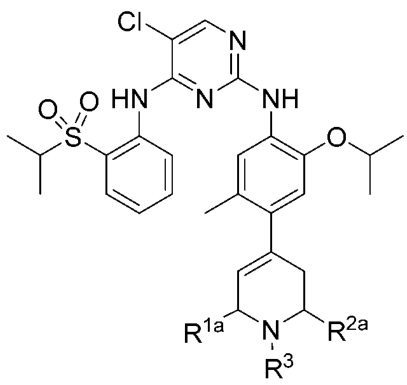

**(I)**

wherein $R^{1a}$ and $R^{2a}$ are independently hydrogen, $C_{1-4}$ alkyl, or $C_{3-6}$ cycloalkyl; $R^3$ is

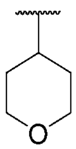

.

**2.** The pharmaceutical combination according to claim 1, wherein one or more of the following conditions are met:

(1) the pharmaceutical combination is a pharmaceutical combination for use in treating and/or preventing ovarian cancer;
(2) the substance X is in a therapeutically effective amount;
(3) the substance Y is in a therapeutically effective amount;
(4) the substance X is the compound represented by formula (I) or the pharmaceutically acceptable salt thereof, for example, the compound represented by formula (I); preferably, the compound represented by formula (I) is a compound represented by formula (I-1):

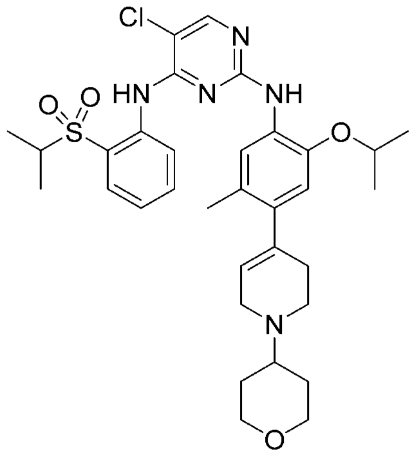

(I-1) ;

(5) the substance Y is doxorubicin or the pharmaceutically acceptable salt thereof, for example, the pharmaceutically acceptable salt of doxorubicin, preferably doxorubicin hydrochloride or PLD; and
(6) a mass ratio of the substance X to the substance Y within one administration cycle of 28 days is (200-1000):1, preferably (300-900):1, and more preferably (300-800):1.

**3.** The pharmaceutical combination according to claim 1, wherein the substance X is a compound represented by formula (I-1), the substance Y is doxorubicin hydrochloride or PLD, and a mass ratio of the substance X to the substance Y within one administration cycle of 28 days is (200-1000):1, preferably (300-900):1, and more preferably (300-800):1.

**4.** The pharmaceutical combination according to claim 1, wherein one or more of the following conditions are met:

(1) the substance X is administered at a dose of 50 mg/kg to 200 mg/kg, for example, 80 mg/kg to 120 mg/kg, preferably 100 mg/kg; or the substance X is administered at a dose of 600 mg to 2000 mg, preferably 900 mg to 1500 mg, and more preferably 1200 mg, wherein the dose may be in the form of one dose or a plurality of doses;
(2) the substance X is administered once daily;
(3) the substance X is administered by intragastric administration or oral administration, for example, intragastric administration;
(4) the substance Y is administered at a dose of 1 mg/kg to 15 mg/kg, for example, 2 mg/kg to 10 mg/kg, preferably 3 mg/kg; or the substance Y is administered by intravenous drip infusion at a dose of 40 $mg/m^2$, for example, 40 mg to 80 mg, such as 50 mg to 70 mg, wherein the dose may be in the form of one dose or a plurality of doses;
(5) the substance Y is administered once weekly or once every 28 days;
(6) the substance Y is administered by intravenous injection, for example, by intravenous bolus injection; and
(7) the substance X and the substance Y are used simultaneously, separately, or sequentially.

**5.** The pharmaceutical combination according to at least one of claims 1 to 4, wherein the substance X is administered once daily, the substance Y is administered once weekly in a three-week cycle, or the substance Y is administered once every 28 days.

**6.** The pharmaceutical combination according to claim 5, wherein the substance X is administered once daily at a dose of 100 mg/kg or 600 mg to 2000 mg, and the substance Y is administered once weekly in a three-week cycle at a dose of 3 mg/kg; or the substance Y is administered once every 28 days at a dose of 40 mg to 80 mg.

**7.** The pharmaceutical combination according to claim 5, wherein the substance X is administered once daily at a dose of 100 mg/kg or 600 mg to 2000 mg by intragastric administration or oral administration; the substance Y is administered once weekly in a three-week cycle at a dose of 3 mg/kg by intravenous bolus injection; or the substance Y is administered once every 28 days at a dose of 40 mg to 80 mg by intravenous bolus injection.

**8.** The pharmaceutical combination according to claim 5, wherein the substance X is the compound represented by formula (I-1), and the substance X is administered once daily at a dose of 100 mg/kg or 600 mg to 2000 mg by intragastric administration or oral administration; the substance Y is doxorubicin hydrochloride or PLD, and the

substance Y is administered once weekly in a three-week cycle at a dose of 3 mg/kg by intravenous bolus injection; or the substance Y is administered once every 28 days at a dose of 40 mg to 80 mg by intravenous bolus injection.

**9.** A pharmaceutical composition A, comprising:

a first pharmaceutical composition comprising a substance X and a pharmaceutically acceptable excipient, wherein the substance X is the compound represented by formula (I), the pharmaceutically acceptable salt thereof, the solvate thereof, or the solvate of the pharmaceutically acceptable salt thereof according to claim 1; and
a second pharmaceutical composition comprising a substance Y and a pharmaceutically acceptable excipient, wherein the substance Y is doxorubicin, a pharmaceutically acceptable salt thereof, a solvate thereof, or a solvate of the pharmaceutically acceptable salt thereof.

**10.** The pharmaceutical composition A according to claim 9, wherein one or more of the following conditions are met:

(1) the pharmaceutical composition A is a pharmaceutical composition A for use in treating and/or preventing ovarian cancer;
(2) in the first pharmaceutical composition, the substance X is in a therapeutically effective amount;
(3) in the second pharmaceutical composition, the substance Y is in a therapeutically effective amount;
(4) in the first pharmaceutical composition, the substance X is the compound represented by formula (I) or the pharmaceutically acceptable salt thereof, for example, the compound represented by formula (I); preferably, the compound represented by formula (I) is a compound represented by formula (I-1):

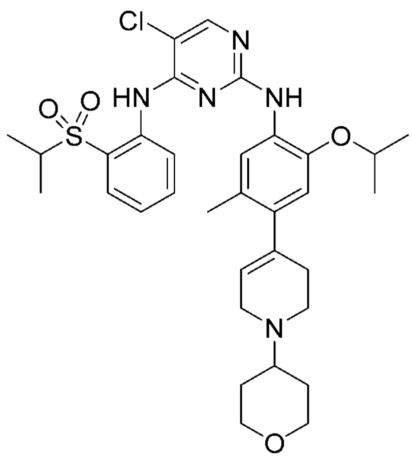

**(I-1)** ;

and
(5) in the second pharmaceutical composition, the substance Y is doxorubicin or the pharmaceutically acceptable salt thereof, for example, the pharmaceutically acceptable salt of doxorubicin, preferably doxorubicin hydrochloride.

**11.** The pharmaceutical composition A according to claim 9, wherein the second pharmaceutical composition is a substance Y liposome.

**12.** The pharmaceutical composition A according to claim 9, wherein one or more of the following conditions are met:

(1) in the first pharmaceutical composition, the substance X is administered at a dose of 50 mg/kg to 200 mg/kg, for example, 80 mg/kg to 120 mg/kg, preferably 100 mg/kg; or the substance X is administered at a dose of 600 mg to 2000 mg, preferably 900 mg to 1500 mg, and more preferably 1200 mg, wherein the dose may be in the form of one dose or a plurality of doses;
(2) the first pharmaceutical composition is administered once daily;
(3) the first pharmaceutical composition is administered by intragastric administration or oral administration;
(4) the second pharmaceutical composition is a substance Y liposome, and the substance Y liposome is administered at a dose of 1 mg/kg to 15 mg/kg, for example, 2 mg/kg to 10 mg/kg, preferably 3 mg/kg; or the

substance Y is administered by intravenous drip infusion at a dose of 40 $mg/m^2$, for example, 40 mg to 80 mg, such as 50 mg to 70 mg, wherein the dose may be in the form of one dose or a plurality of doses;
(5) the second pharmaceutical composition is administered once weekly or once every 28 days;
(6) the second pharmaceutical composition is administered by intravenous injection, for example, by intravenous bolus injection;
(7) the first pharmaceutical composition and the second pharmaceutical composition are administered simultaneously, separately, or sequentially; and
(8) the second pharmaceutical composition is pegylated liposomal doxorubicin.

**13.** The pharmaceutical composition A according to claim 9, wherein the second pharmaceutical composition is a substance Y liposome, and a mass ratio of the substance X to the substance Y liposome in the first pharmaceutical composition within one administration cycle of 28 days is (200-1000):1, preferably (300-900):1, and more preferably (300-800):1.

**14.** The pharmaceutical composition A according to claim 9, comprising:

the first pharmaceutical composition comprising the substance X and the pharmaceutically acceptable excipient, the substance X being a compound represented by formula (I-1); and
the second pharmaceutical composition,
the second pharmaceutical composition being pegylated liposomal doxorubicin,
wherein a mass ratio of the compound represented by formula (I-1) to the pegylated liposomal doxorubicin within one administration cycle of 28 days is (200-1000):1, preferably (300-900): 1, and more preferably (300-800):1.

**15.** The pharmaceutical composition A according to at least one of claims 9 to 14, wherein the first pharmaceutical composition is administered once daily, and the second pharmaceutical composition is administered once weekly in a three-week cycle or once every 28 days.

**16.** The pharmaceutical composition A according to claim 15, wherein in the first pharmaceutical composition, the substance X is administered at a dose of 100 mg/kg or 600 mg to 2000 mg, and the first pharmaceutical composition is administered once daily; the second pharmaceutical composition is the substance Y liposome, the substance Y liposome is administered at a dose of 3 mg/kg, the second pharmaceutical composition is administered once weekly in a three-week cycle, or the substance Y liposome is administered once every 28 days at a dose of 40 mg to 80 mg.

**17.** The pharmaceutical composition A according to claim 15, wherein in the first pharmaceutical composition, the substance X is administered at a dose of 100 mg/kg or 600 mg to 2000 mg, and the first pharmaceutical composition is administered once daily by intragastric administration or oral administration; the second pharmaceutical composition is the substance Y liposome, the substance Y liposome is administered at a dose of 3 mg/kg or 40 mg to 80 mg, and the second pharmaceutical composition is administered once weekly in a three-week cycle or once every 28 days by intravenous bolus injection.

**18.** The pharmaceutical composition A according to claim 15, wherein in the first pharmaceutical composition, the substance X is the compound represented by formula (I-1), the substance X is administered at a dose of 100 mg/kg or 600 mg to 2000 mg, and the first pharmaceutical composition is administered once daily by intragastric administration or oral administration; the second pharmaceutical composition is pegylated liposomal doxorubicin, the pegylated liposomal doxorubicin is administered at a dose of 3 mg/kg or 40 mg to 80 mg, and the pegylated liposomal doxorubicin is administered once weekly in a three-week cycle or once every 28 days by intravenous bolus injection.

**19.** A combination kit, comprising:

a first container comprising the first pharmaceutical composition according to at least one of claims 9 to 18; and
a second container comprising the second pharmaceutical composition according to at least one of claims 9 to 18.

**20.** Use of the pharmaceutical combination according to at least one of claims 1 to 8 or the pharmaceutical composition A according to at least one of claims 9 to 18 in preparing a medicament for preventing and/or treating ovarian cancer.

**21.** A method for preventing and/or treating ovarian cancer, comprising administering to a patient in need thereof the pharmaceutical combination according to at least one of claims 1 to 8 or the pharmaceutical composition A according to at least one of claims 9 to 18.

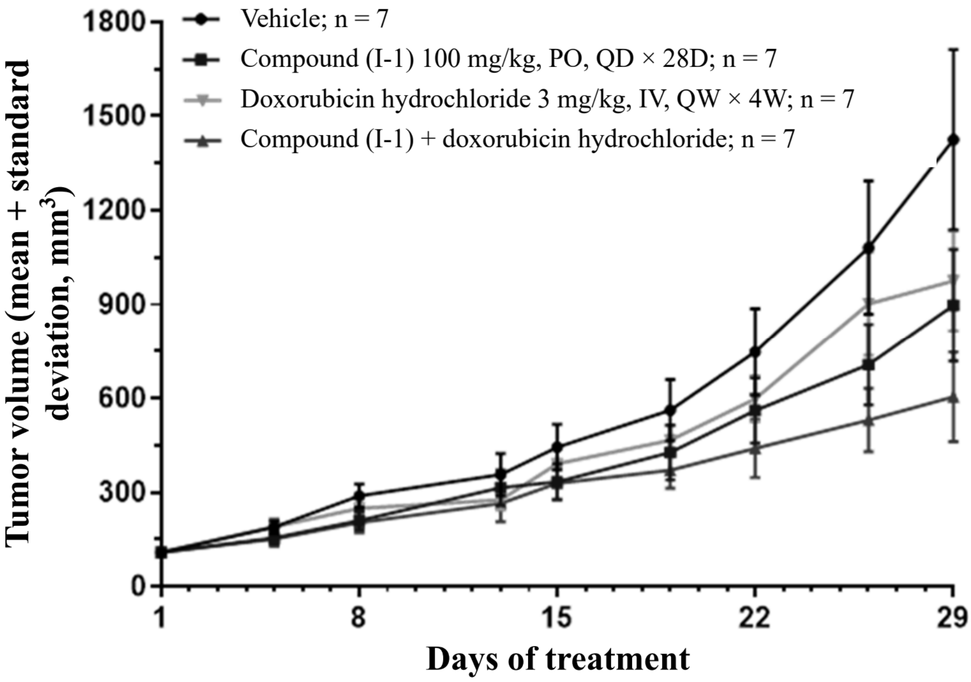
Vehicle; n = 7
Compound (I-1) 100 mg/kg, PO, QD × 28D; n = 7
Doxorubicin hydrochloride 3 mg/kg, IV, QW × 4W; n = 7
Compound (I-1) + doxorubicin hydrochloride; n = 7
Tumor volume (mean + standard deviation, mm3)
0
300
600
900
1200
1500
1800
1
8
15
22
29
Days of treatment

FIG. 1

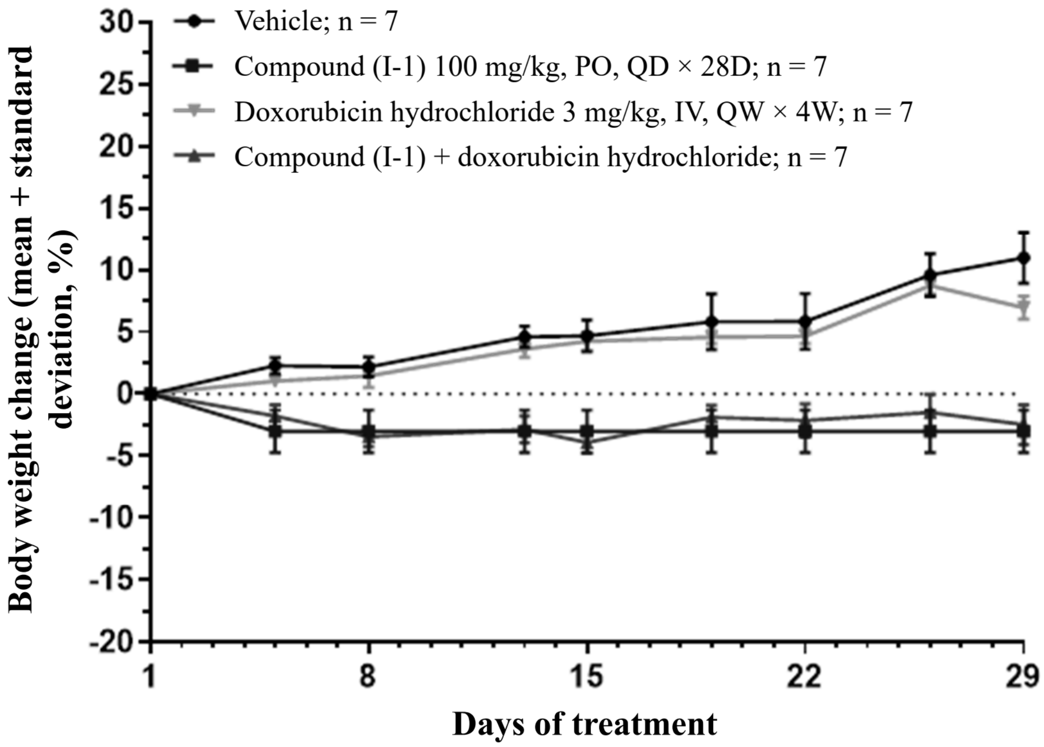
Vehicle; n = 7
Compound (I-1) 100 mg/kg, PO, QD × 28D; n = 7
Doxorubicin hydrochloride 3 mg/kg, IV, QW × 4W; n = 7
Compound (I-1) + doxorubicin hydrochloride; n = 7
Body weight change (mean + standard deviation, %)
30
25
20
15
10
5
0
-5
-10
-15
-20
1
8
15
22
29
Days of treatment

FIG. 2

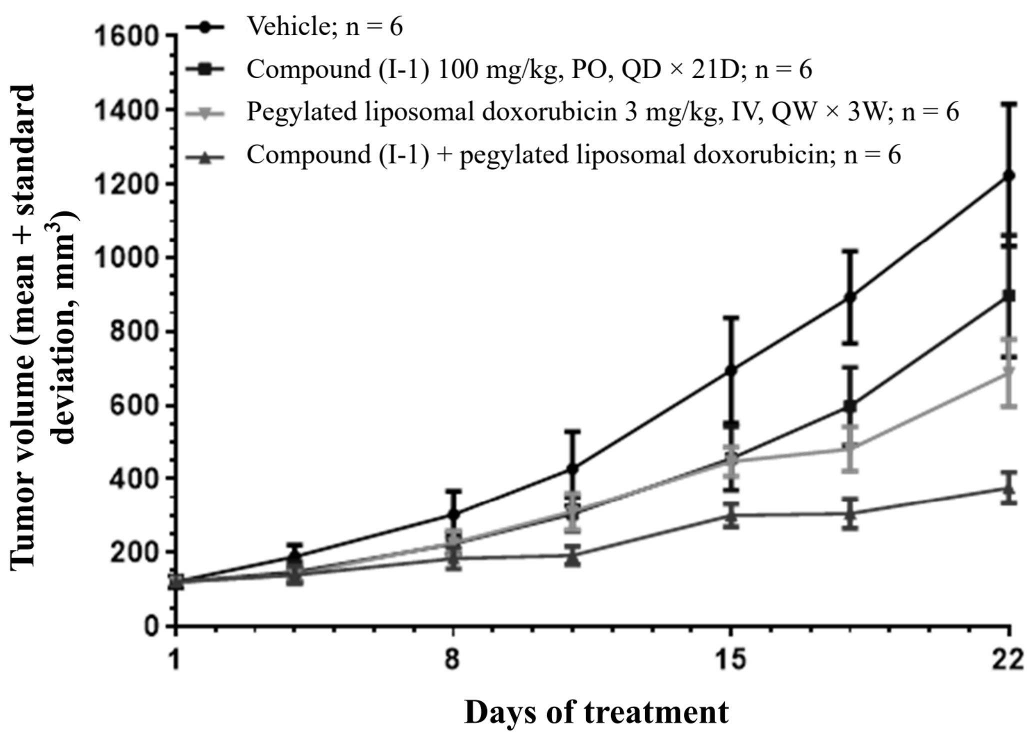
Vehicle; n = 6
Compound (I-1) 100 mg/kg, PO, QD × 21D; n = 6
Pegylated liposomal doxorubicin 3 mg/kg, IV, QW × 3W; n = 6
Compound (I-1) + pegylated liposomal doxorubicin; n = 6
Tumor volume (mean + standard deviation, mm³)
1600
1400
1200
1000
800
600
400
200
0
1
8
15
22
Days of treatment

FIG. 3

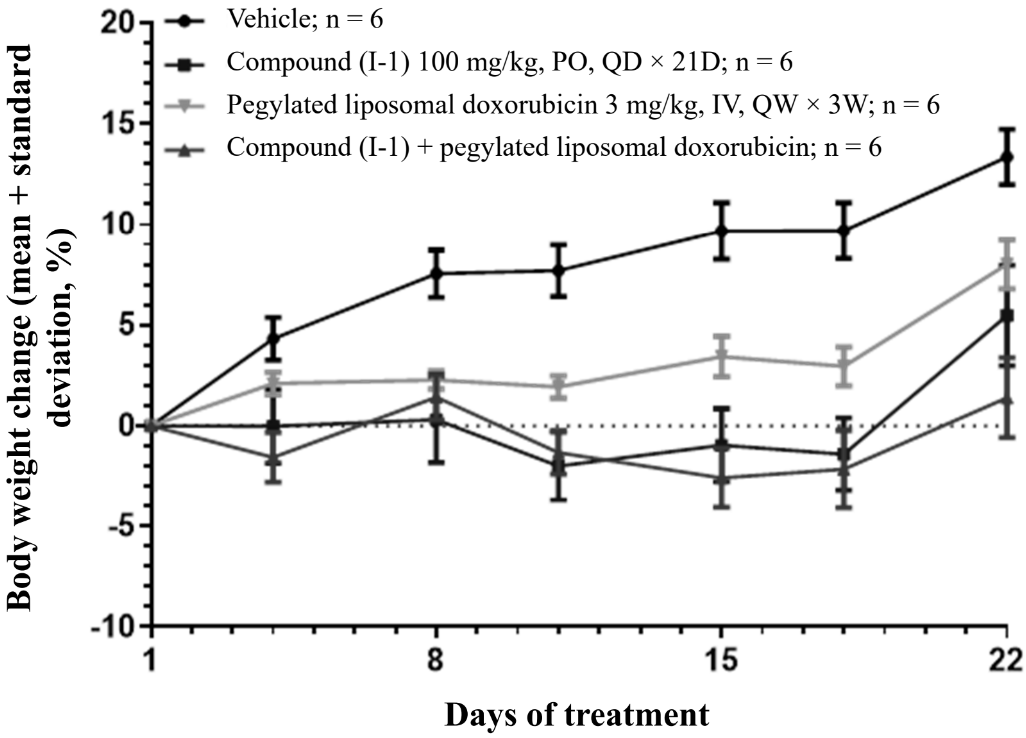
Vehicle; n = 6
Compound (I-1) 100 mg/kg, PO, QD × 21D; n = 6
Pegylated liposomal doxorubicin 3 mg/kg, IV, QW × 3W; n = 6
Compound (I-1) + pegylated liposomal doxorubicin; n = 6
Body weight change (mean + standard deviation, %)
20
15
10
5
0
-5
-10
1
8
15
22
Days of treatment

FIG. 4

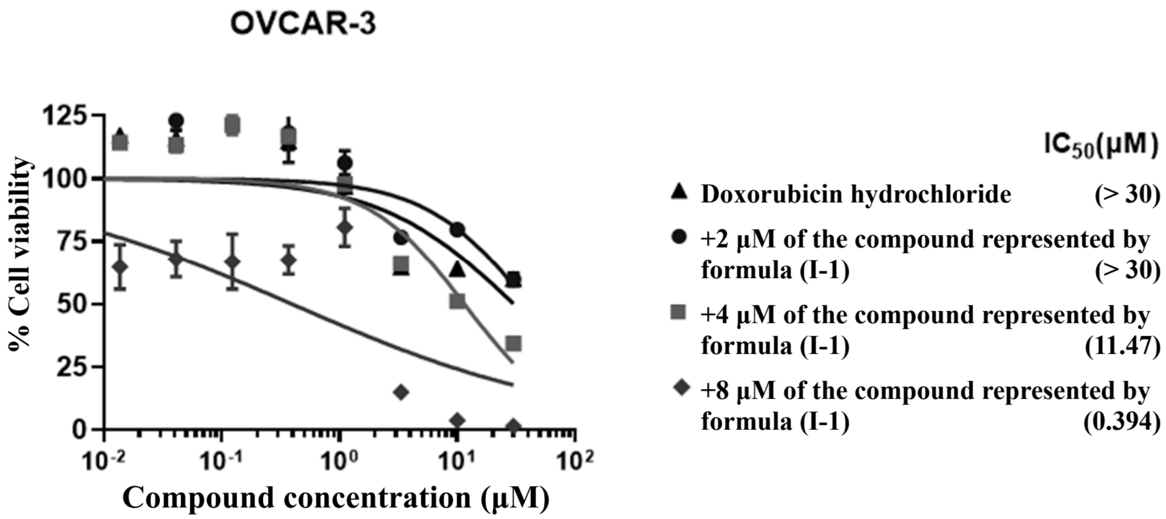
OVCAR-3
% Cell viability
125
100
75
50
25
0
$10^{-2}$
$10^{-1}$
$10^{0}$
$10^{1}$
$10^{2}$
Compound concentration (μM)
$IC_{50}$(μM)
Doxorubicin hydrochloride (> 30)
+2 μM of the compound represented by formula (I-1) (> 30)
+4 μM of the compound represented by formula (I-1) (11.47)
+8 μM of the compound represented by formula (I-1) (0.394)

FIG. 5

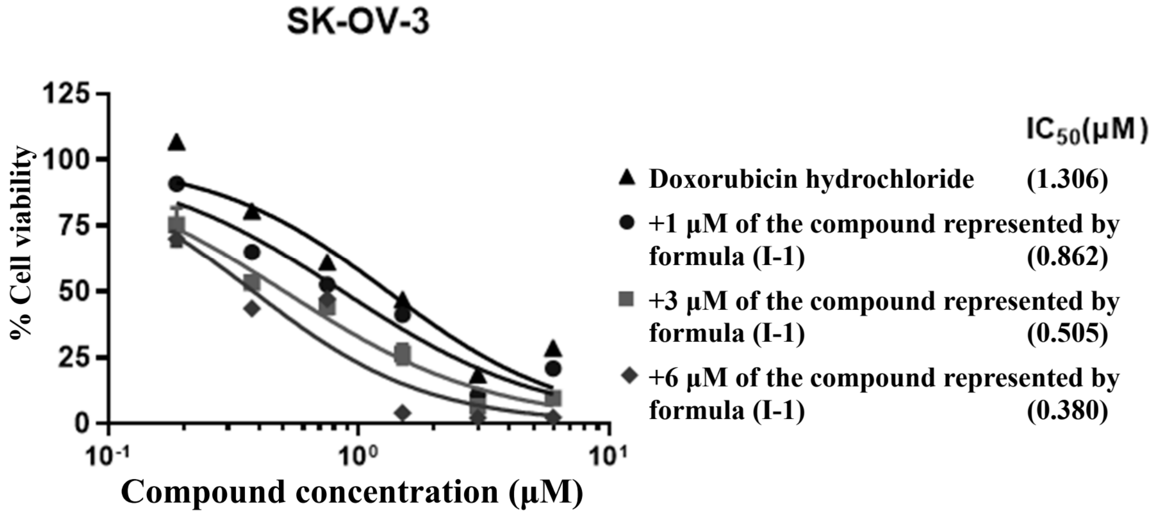
SK-OV-3
125
100
75
50
25
0
% Cell viability
10-1
100
101
Compound concentration (µM)
IC50(µM)
Doxorubicin hydrochloride (1.306)
+1 µM of the compound represented by formula (I-1) (0.862)
+3 µM of the compound represented by formula (I-1) (0.505)
+6 µM of the compound represented by formula (I-1) (0.380)

FIG. 6

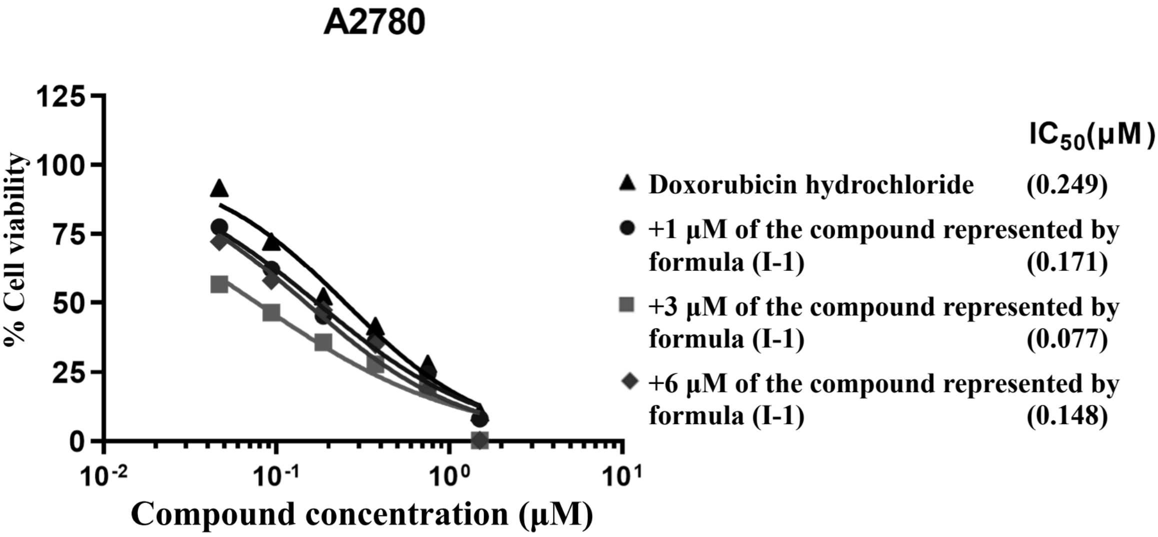
A2780
125
100
75
50
25
0
% Cell viability
10-2
10-1
100
101
Compound concentration (µM)
IC50(µM)
Doxorubicin hydrochloride (0.249)
+1 µM of the compound represented by formula (I-1) (0.171)
+3 µM of the compound represented by formula (I-1) (0.077)
+6 µM of the compound represented by formula (I-1) (0.148)

FIG. 7

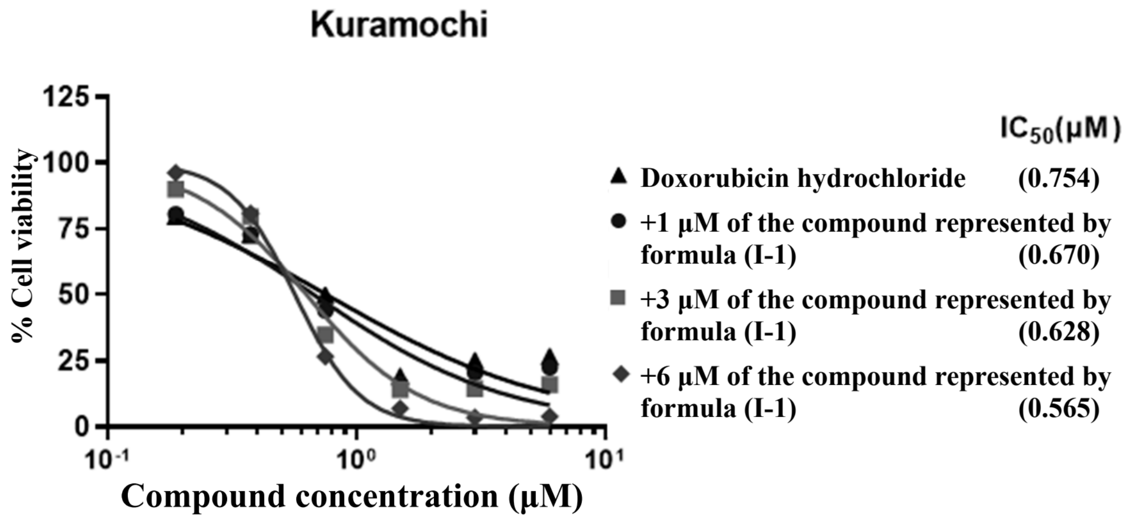
Kuramochi
125
100
75
50
25
0
% Cell viability
10⁻¹
10⁰
10¹
Compound concentration (μM)
IC50(μM)
Doxorubicin hydrochloride (0.754)
+1 μM of the compound represented by formula (I-1) (0.670)
+3 μM of the compound represented by formula (I-1) (0.628)
+6 μM of the compound represented by formula (I-1) (0.565)

FIG. 8

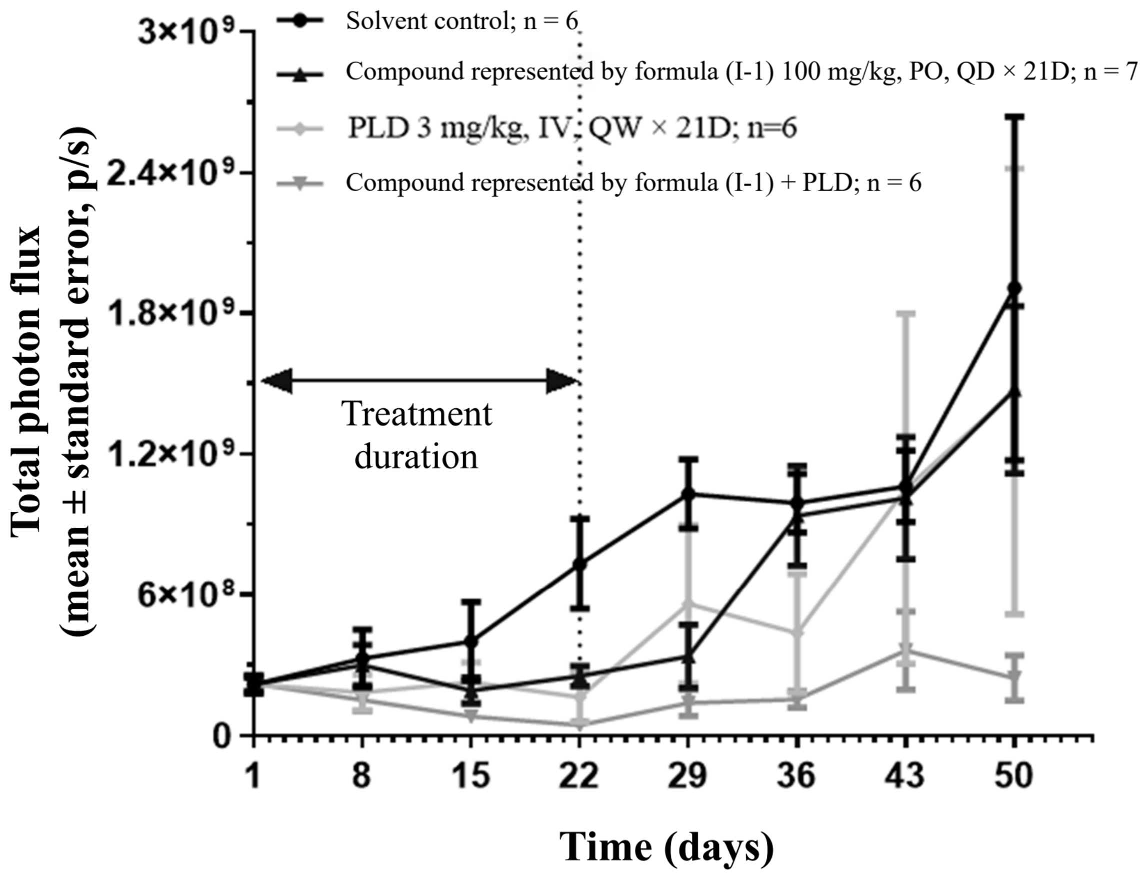
Solvent control; n = 6
Compound represented by formula (I-1) 100 mg/kg, PO, QD × 21D; n = 7
PLD 3 mg/kg, IV, QW × 21D; n=6
Compound represented by formula (I-1) + PLD; n = 6
3×10⁹
2.4×10⁹
1.8×10⁹
1.2×10⁹
6×10⁸
0
Total photon flux
(mean ± standard error, p/s)
Treatment
duration
1
8
15
22
29
36
43
50
Time (days)

FIG. 9

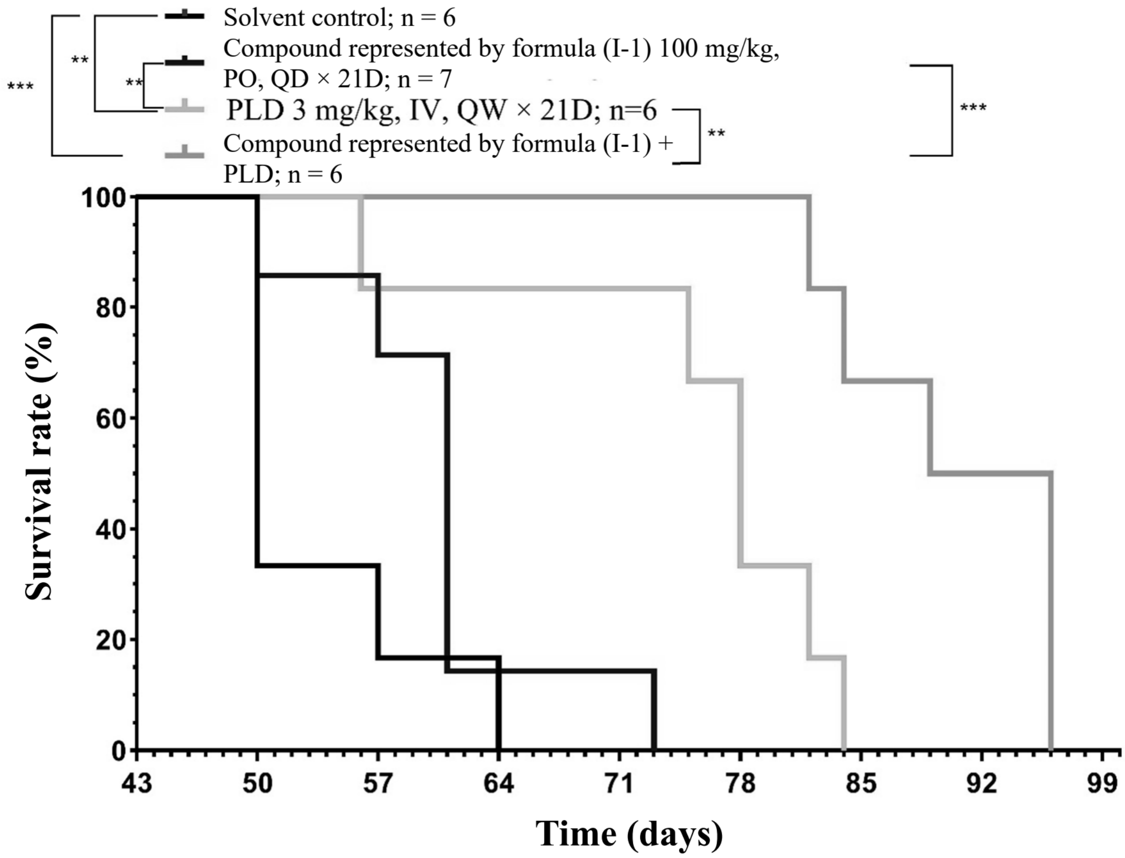
Solvent control; n = 6
Compound represented by formula (I-1) 100 mg/kg, PO, QD × 21D; n = 7
PLD 3 mg/kg, IV, QW × 21D; n=6
Compound represented by formula (I-1) + PLD; n = 6
***
**
**
***
**
Survival rate (%)
100
80
60
40
20
0
43
50
57
64
71
78
85
92
99
Time (days)

FIG. 10

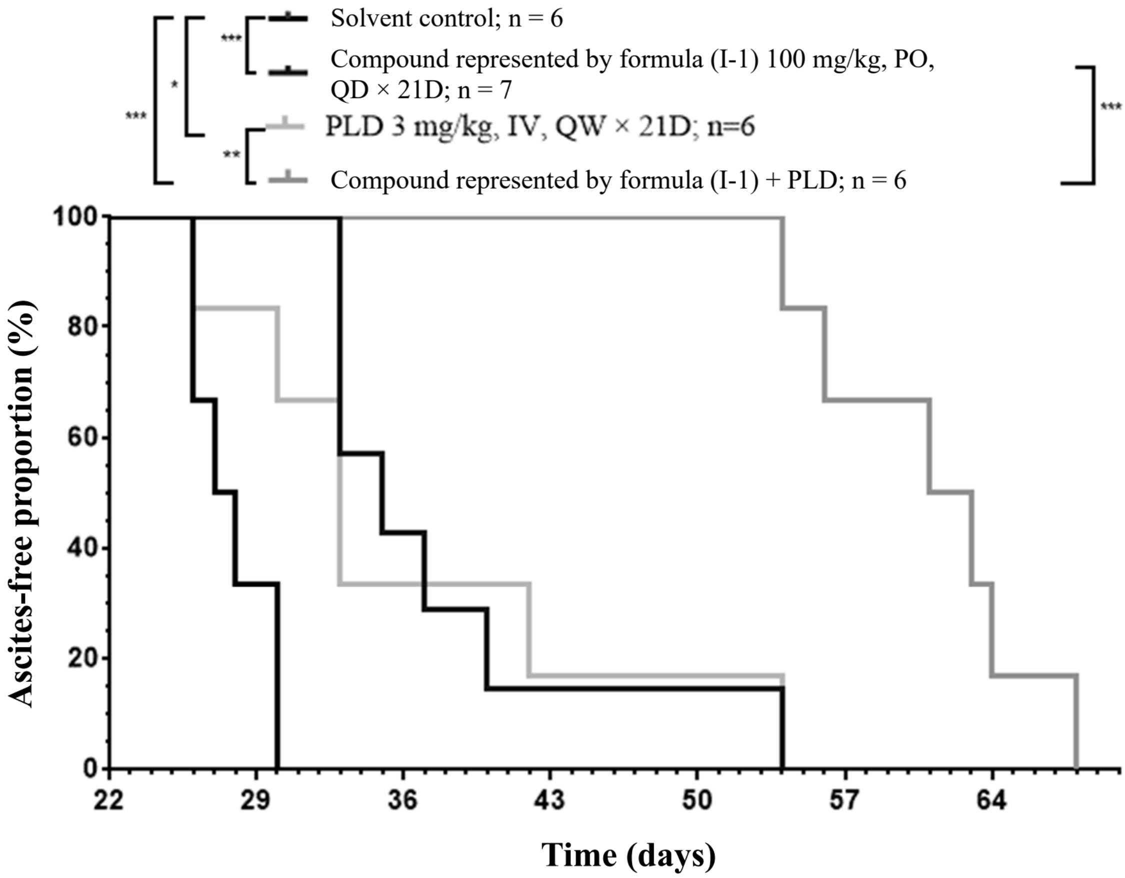
Solvent control; n = 6
Compound represented by formula (I-1) 100 mg/kg, PO, QD × 21D; n = 7
PLD 3 mg/kg, IV, QW × 21D; n=6
Compound represented by formula (I-1) + PLD; n = 6
***
*
***
**
***
Ascites-free proportion (%)
100
80
60
40
20
0
22
29
36
43
50
57
64
Time (days)

FIG. 11

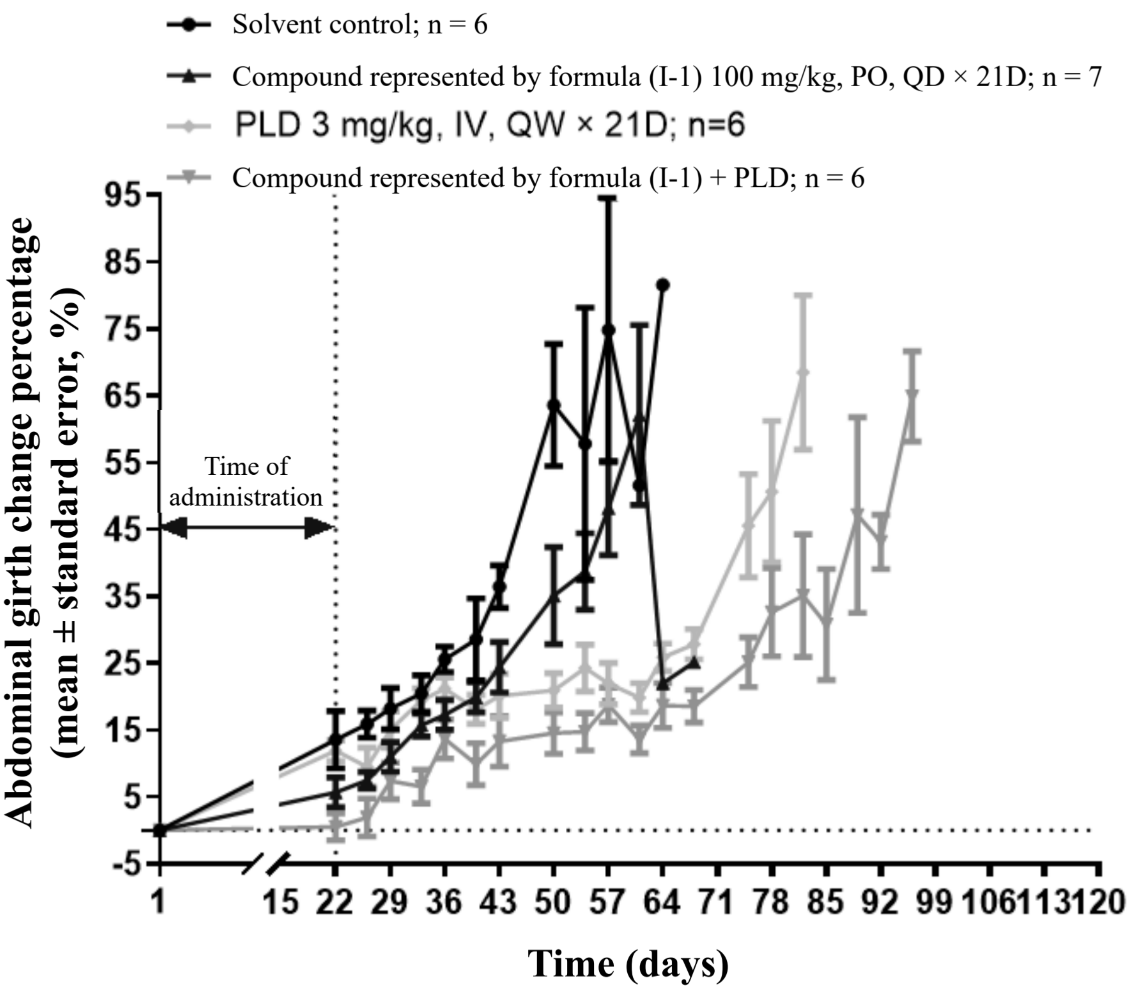
Solvent control; n = 6
Compound represented by formula (I-1) 100 mg/kg, PO, QD × 21D; n = 7
PLD 3 mg/kg, IV, QW × 21D; n=6
Compound represented by formula (I-1) + PLD; n = 6
Abdominal girth change percentage (mean ± standard error, %)
95
85
75
65
55
45
35
25
15
5
-5
Time of administration
1
15
22
29
36
43
50
57
64
71
78
85
92
99
106
113
120
Time (days)

FIG. 12

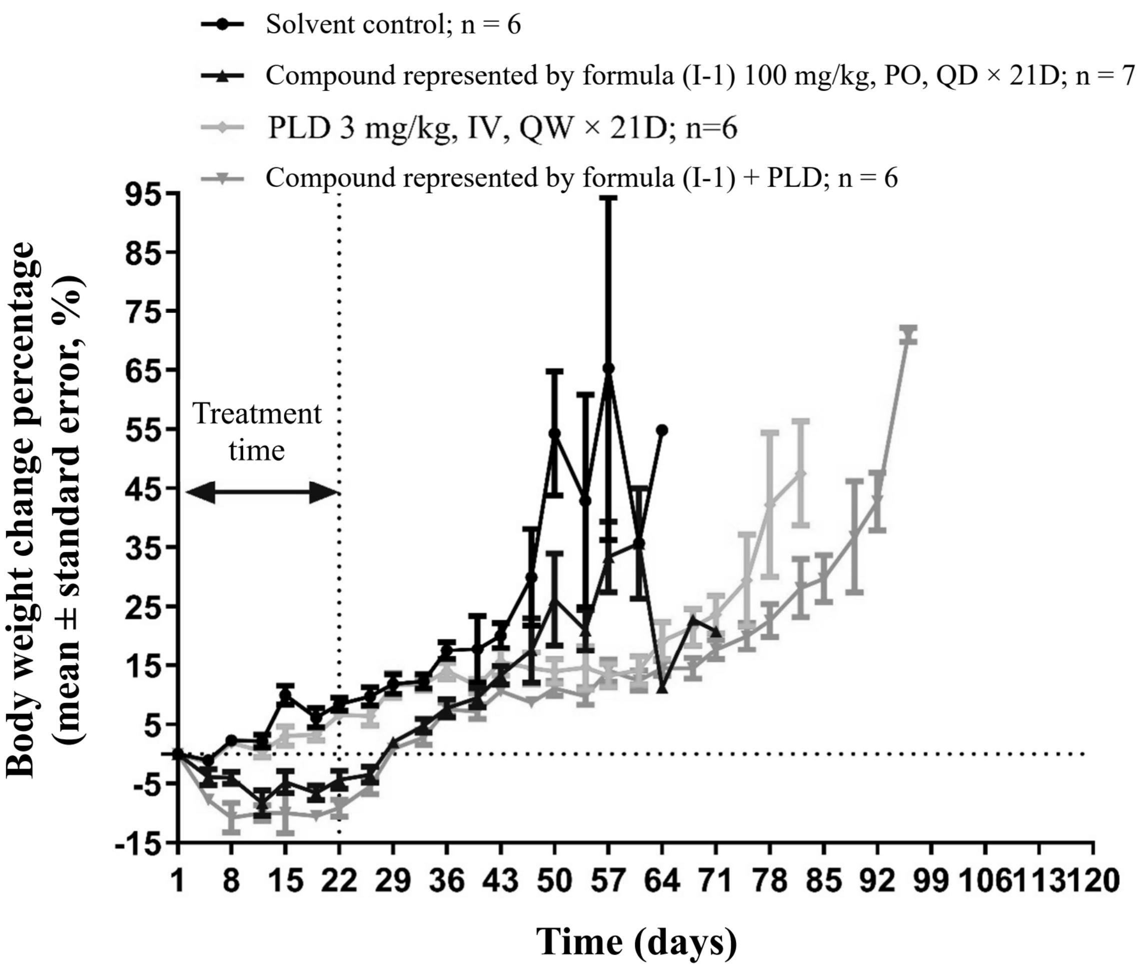
Solvent control; n = 6
Compound represented by formula (I-1) 100 mg/kg, PO, QD × 21D; n = 7
PLD 3 mg/kg, IV, QW × 21D; n=6
Compound represented by formula (I-1) + PLD; n = 6
Body weight change percentage (mean ± standard error, %)
95
85
75
65
55
45
35
25
15
5
-5
-15
Treatment time
1
8
15
22
29
36
43
50
57
64
71
78
85
92
99
106
113
120
Time (days)

FIG. 13

**INTERNATIONAL SEARCH REPORT**

International application No.
**PCT/CN2024/083301**

**A. CLASSIFICATION OF SUBJECT MATTER**

A61K 31/704(2006.01)i; A61K31/506(2006.01)i; C07D405/14(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07D, A61P

Documentation searched other than minimum documentation to the extent that such documents are included in the fields searched

Electronic data base consulted during the international search (name of data base and, where practicable, search terms used)

CNABS; CNTXT; DWPI; ENTXT; CNKI; STNext REGISTRY/CAPLUS: 基于式(I)的结构检索, structure search based on formula (I), ALK抑制剂, 克唑替尼, 色瑞替尼, 阿霉素, 多柔比星, 联合用药, 卵巢癌, ALK inhibitors, Crizotinib, Ceritinib, Doxorubicin, combination, ovarian cancer

**C. DOCUMENTS CONSIDERED TO BE RELEVANT**

| Category* | Citation of document, with indication, where appropriate, of the relevant passages | Relevant to claim No. |
|---|---|---|
| X | CN 109715620 A (THE REGENTS OF THE UNIVERSITY OF MICHIGAN) 03 May 2019 (2019-05-03) table 1, compound 5, and description, paragraphs 0109 and 0151 | 1-21 |
| Y | CN 109715620 A (THE REGENTS OF THE UNIVERSITY OF MICHIGAN) 03 May 2019 (2019-05-03) table 1, compound 5, and description, paragraphs 0109 and 0151 | 1-21 |
| Y | CN 107320474 A (ZHEJIANG UNIVERSITY) 07 November 2017 (2017-11-07) claims 1-9 | 1-21 |
| Y | CN 112870194 A (AFFILIATED CANCER HOSPITAL OF GUANGZHOU MEDICAL UNIVERSITY) 01 June 2021 (2021-06-01) claims 1-10 | 1-21 |
| Y | CN 114901289 A (UNIVERSITETET I OSLO) 12 August 2022 (2022-08-12) description, paragraphs 0182 and 0247 | 1-21 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **13 June 2024** | **19 June 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/083301**

**C. DOCUMENTS CONSIDERED TO BE RELEVANT**

| Category* | Citation of document, with indication, where appropriate, of the relevant passages | Relevant to claim No. |
|---|---|---|
| Y | HU, Jing et al. "Effect of Ceritinib (LDK378) on Enhancement of Chemotherapeutic Agents in ABCB1 and ABCG2 Overexpressing Cells In Vitro and In Vivo" *Oncotarget,* Vol. 6, No. (42), 09 November 2015 (2015-11-09), 44643-44659<br>abstract | 1-21 |

Form PCT/ISA/210 (second sheet) (July 2022)

**INTERNATIONAL SEARCH REPORT**

International application No.
**PCT/CN2024/083301**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21**
because they relate to subject matter not required to be searched by this Authority, namely:

Claim 21 relates to a method for preventing and/or treating ovarian cancer, and therefore does not comply with PCT Rule 39.1(iv). A search is provided on the basis of the pharmaceutical use of the drug combination in claim 21.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/083301**

| Patent document cited in search report | | | Publication date (day/month/year) | Patent family member(s) | | | Publication date (day/month/year) |
|---|---|---|---|---|---|---|---|
| CN | 109715620 | A | 03 May 2019 | AU | 2017319135 | A1 | 04 April 2019 |
| | | | | AU | 2017319135 | B2 | 18 March 2021 |
| | | | | NZ | 751713 | A | 01 July 2022 |
| | | | | EP | 4001273 | A2 | 25 May 2022 |
| | | | | EP | 4001273 | A3 | 24 August 2022 |
| | | | | AU | 2021203098 | A1 | 10 June 2021 |
| | | | | AU | 2021203098 | B2 | 25 May 2023 |
| | | | | US | 2020330464 | A1 | 22 October 2020 |
| | | | | US | 11110090 | B2 | 07 September 2021 |
| | | | | IL | 264638 | B | 31 August 2021 |
| | | | | JP | 2022120151 | A | 17 August 2022 |
| | | | | US | 2019175595 | A1 | 13 June 2019 |
| | | | | US | 10709705 | B2 | 14 July 2020 |
| | | | | BR | 112019003897 | A2 | 21 May 2019 |
| | | | | JP | 2019528307 | A | 10 October 2019 |
| | | | | JP | 7094566 | B2 | 04 July 2022 |
| | | | | KR | 20190039760 | A | 15 April 2019 |
| | | | | KR | 102530871 | B1 | 09 May 2023 |
| | | | | SG | 10201914030 | UA | 30 March 2020 |
| | | | | MX | 2019002393 | A | 08 July 2019 |
| | | | | CA | 3033223 | A1 | 08 March 2018 |
| | | | | EP | 3504203 | A2 | 03 July 2019 |
| | | | | EP | 3504203 | B1 | 28 September 2022 |
| | | | | MX | 2022000376 | A | 10 February 2022 |
| | | | | SG | 11201901251 | SA | 28 March 2019 |
| | | | | WO | 2018044767 | A2 | 08 March 2018 |
| | | | | WO | 2018044767 | A3 | 12 April 2018 |
| CN | 107320474 | A | 07 November 2017 | | None | | |
| CN | 112870194 | A | 01 June 2021 | | None | | |
| CN | 114901289 | A | 12 August 2022 | JP | 2022554258 | A | 28 December 2022 |
| | | | | KR | 20220098158 | A | 11 July 2022 |
| | | | | EP | 4051288 | A1 | 07 September 2022 |
| | | | | US | 2022401444 | A1 | 22 December 2022 |
| | | | | CA | 3159339 | A1 | 06 May 2021 |
| | | | | GB | 201915618 | D0 | 11 December 2019 |
| | | | | AU | 2020374150 | A1 | 09 June 2022 |
| | | | | WO | 2021083555 | A1 | 06 May 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BERGE et al.** Pharmaceutical Salts. *Journal of Pharmaceutical Science*, 1977, vol. 66, 1-19
- Handbook of Pharmaceutical Salts: Properties, Selection, and Use. Wiley-VCH, 2002
- Pharmacopoeia of the People's Republic of China. 2020, vol. IV
- **RAYMOND C ROWE**. Handbook of Pharmaceutical Excipients. 2009
- Technical Guidelines for Non-Clinical Studies of Cytotoxic Antitumor Drugs. China Food and Drug Administration (CFDA), 2006
- Technical Guidelines for Non-Clinical Studies of Cytotoxic Antitumor Drugs. CFDA, November 2006